# EUROPEAN PATENT SPECIFICATION

(11) **EP 2 121 988 B1**
(45) Date of publication and mention of the grant of the patent: **17.12.2014**
(21) Application number: 08730593.4
(22) Date of filing: 25.02.2008
(51) Int. Cl.: C12Q 1/68

(54) **PROSTATE CANCER SURVIVAL AND RECURRENCE**
ÜBERLEBEN UND REZIDIV VON PROSTATAKREBS
SURVIE AU CANCER DE LA PROSTATE ET RÉCURRENCE DE CE DERNIER

(30) Priority: 23.02.2007 US 891477 P
(43) Date of publication of application: 25.11.2009
(60) Divisional application: 13153098.2
(73) Proprietor: bioTheranostics, Inc., San Diego, CA 92121 (US); General Hospital Corporation, Boston, MA 02114 (US)
(72) Inventor: MA, Xiao-Jun, San Diego, CA 92130 (US); WU, Chin-Lee, Newton, MA 02459 (US); ERLANDER, Mark, G., Carlsbad, CA 92011 (US)
(74) Representative: Roques, Sarah Elizabeth
(86) International application number: PCT/US2008/054820
(87) International publication number: WO 2008/103971

(56) References cited:
- WO-A2-03/053223
- US-A1- 2004 016 006
- US-A1- 2004 253 256
- US-A1- 2006 211 017
- BAR-SHIRA ANAT ET AL: "Multiple genes in human 20q13 chromosomal region are involved in an advanced prostate cancer xenograft." CANCER RESEARCH, vol. 62, no. 23, 1 December 2002 (2002-12-01), pages 6803-6807, XP007912857 ISSN: 0008-5472
- GOLUB T R ET AL: "MOLECULAR CLASSIFICATION OF CANCER: CLASS DISCOVERY AND CLASS PREDICTION BY GENE EXPRESSION MONITORING" SCIENCE, AMERICAN ASSOCIATION FOR THE ADVANCEMENT OF SCIENCE, WASHINGTON, DC; US LNKD- DOI:10.1126/SCIENCE.286.5439.531, vol. 286, 15 October 1999 (1999-10-15), pages 531-537, XP002943419 ISSN: 0036-8075
- SU ANDREW I ET AL: "MOLECULAR CLASSIFICATION OF HUMAN CARCINOMAS BY USE OF GENE EXPRESSION SIGNATURES" CANCER RESEARCH, AMERICAN ASSOCIATION FOR CANCER REREARCH, US, vol. 61, 15 October 2001 (2001-10-15), pages 7388-7393, XP008069516 ISSN: 0008-5472
- PATRIKAINEN L ET AL: "Expression profiling of PC-3 cell line variants and comparison of MIC-I transcript levels in benign and malignant prostate" EUROPEAN JOURNAL OF CLINICAL INVESTIGATION, vol. 37, no. 2, 4 January 2007 (2007-01-04), pages 126-133, XP007912858 ISSN: 0014-2972
- ROSNER ET AL: "Higher Tumor to Benign Ratio of the Androgen Receptor mRNA Expression Associates with Prostate Cancer Progression after Radical Prostatectomy" UROLOGY, BELLE MEAD, NJ, US LNKD- DOI:10.1016/J.UROLOGY.2007.09.010, vol. 70, no. 6, 23 December 2007 (2007-12-23), pages 1225-1229, XP022398167 ISSN: 0090-4295
- MAURER ET AL.: 'FEV acts as a transcriptional repressor through its DNA-binding ETS demain and alanine-rich domain' ONCOGENE vol. 22, 2003, pages 3319 - 3329, XP008126553
- ANDREW J. STEPHENSON ET AL: 'Integration of gene expression profiling and clinical variables to predict prostate carcinoma recurrence after radical prostatectomy' CANCER vol. 104, no. 2, 15 July 2005, pages 290 - 298, XP055010914 DOI: 10.1002/cncr.21157 ISSN: 0008-543X
- DATABASE MEDLINE [Online] US NATIONAL LIBRARY OF MEDICINE (NLM), BETHESDA, MD, US 09 April 2013 WU CHIN-LEE ET AL: 'Development and validation of a 32-gene prognostic index for prostate cancer progression.' Database accession no. NLM23533275 & PROCEEDINGS OF THE NATIONAL ACADEMY OF SCIENCES OF THE UNITED STATES OF AMERICA 9 APR 2013, vol. 110, no. 15, 9 April 2013 (2013-04-09), pages 6121-6126, ISSN: 1091-6490

## Description

### FIELD OF THE DISCLOSURE

The disclosure relates to the identification and use of gene (or sequence) expression profiles, or patterns, with clinical relevance to prostate cancer. In particular, the disclosure is based on the identification of genes, or expressed sequences, the expression of which are correlated with patient survival, prostate cancer recurrence, and/or occurrence of prostate cancer metastasis. The gene expression profiles, whether embodied in nucleic acid expression, protein expression, or other expression formats, may be used to predict the survival of subjects afflicted with prostate cancer, predict prostate cancer recurrence, and/or predict occurrence of prostate cancer metastasis. The profiles may also be used in the study and/or diagnosis of prostate cancer cells and tissue as well as for the study and/or determination of prognosis of a patient. When used for diagnosis or prognosis, the profiles are used to determine the treatment of prostate cancer based upon the likelihood of life expectancy, cancer recurrence, and/or cancer metastasis.

### BACKGROUND OF THE DISCLOSURE

Prostate cancer is the most commonly diagnosed cancer in men in the US, with approximately 240,000 newly diagnosed patients and 40,000 cancer death each year. Diagnosing and managing prostate cancer are clinically difficult due to lack of the knowledge of the cancer at molecular and genetic levels and a lack of understanding of the natural disease progression.

Prostate cancer research is limited by an inadequate resource of prostate cancer tissue specimens available for research use, particularly those specimens that are well characterized pathologically, appropriately preserved for RNA and protein studies, and linked with patient clinical, pathologic, and follow-up information.

Surgical treatment is used in treating some cases of prostate cancer. However, a fraction of patients will fail the treatment. This is judged by chemical failure (elevation of PSA after one year of surgery) or development of metastasis (commonly in lymph nodes and bone). The ability to identify, or predict, a post- surgery patient as likely to encounter PSA failure, recurrence of the prostate cancer, and/or development of metastasis, would provide multiple benefits to the patient.

### BRIEF SUMMARY OF THE INVENTION

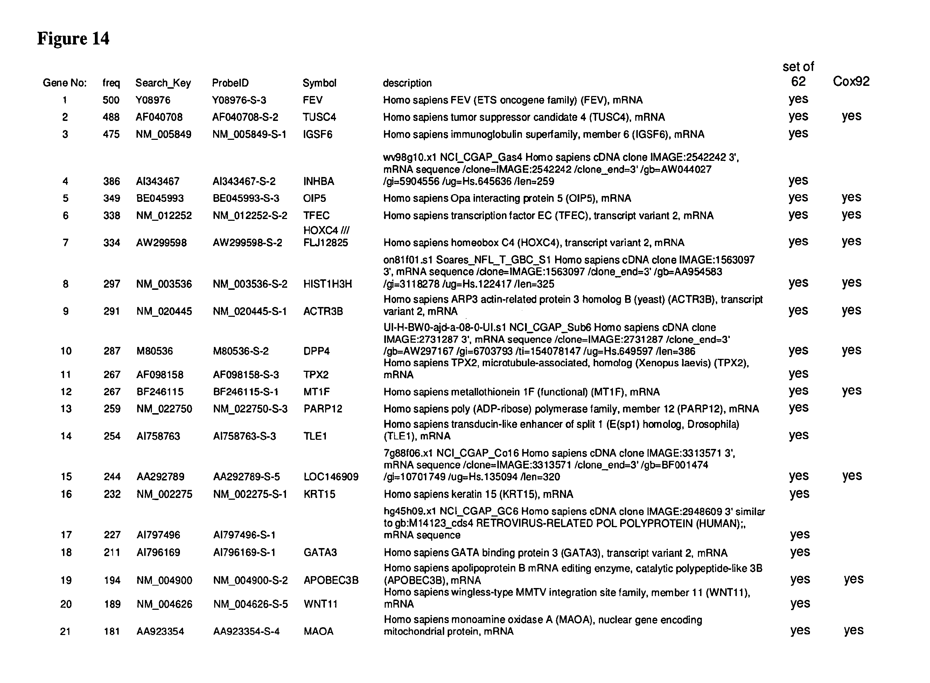

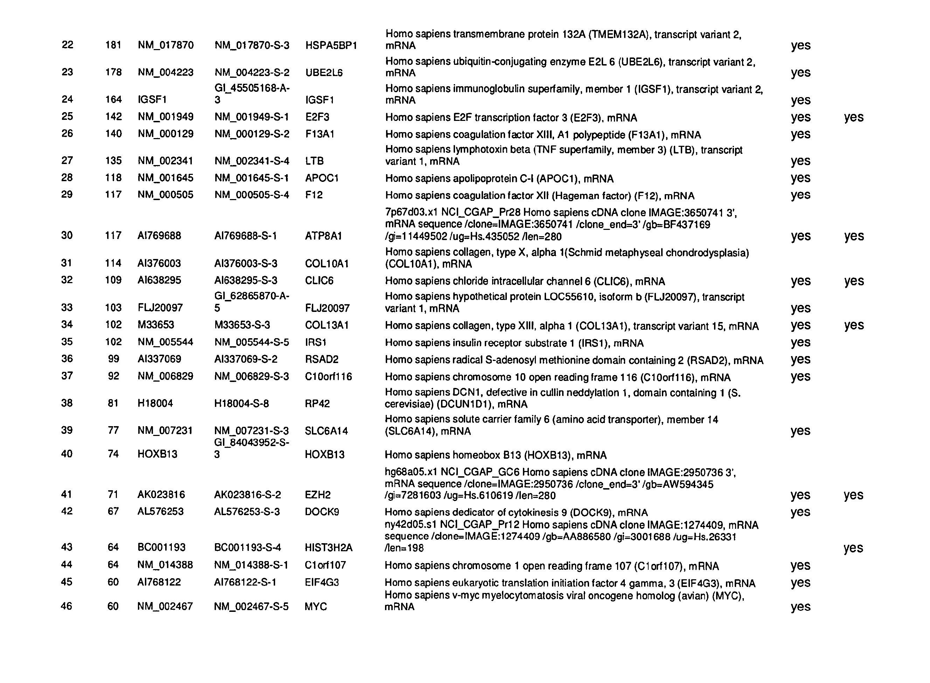

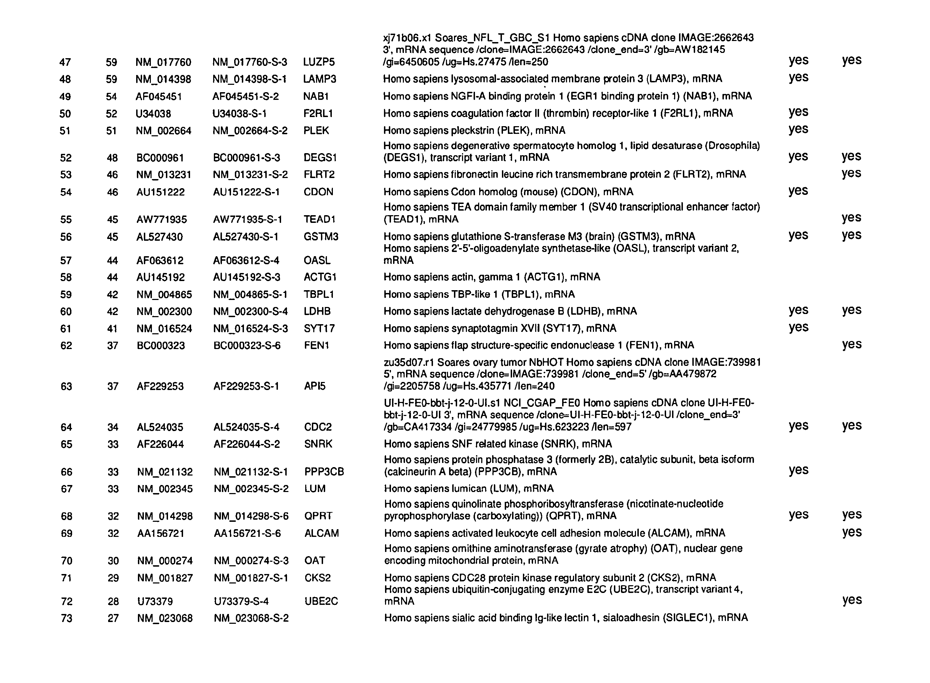

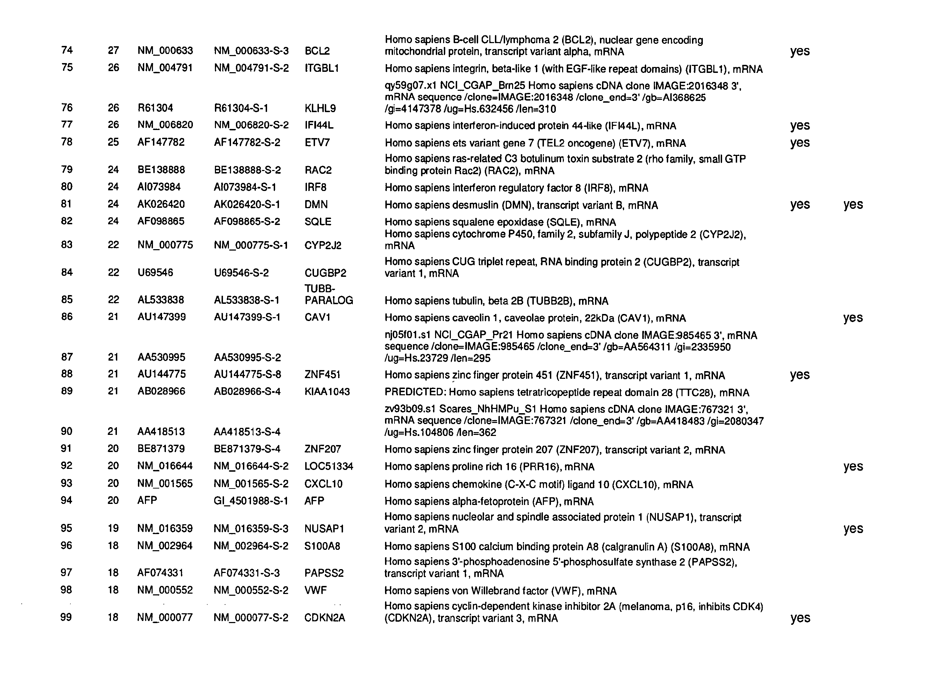

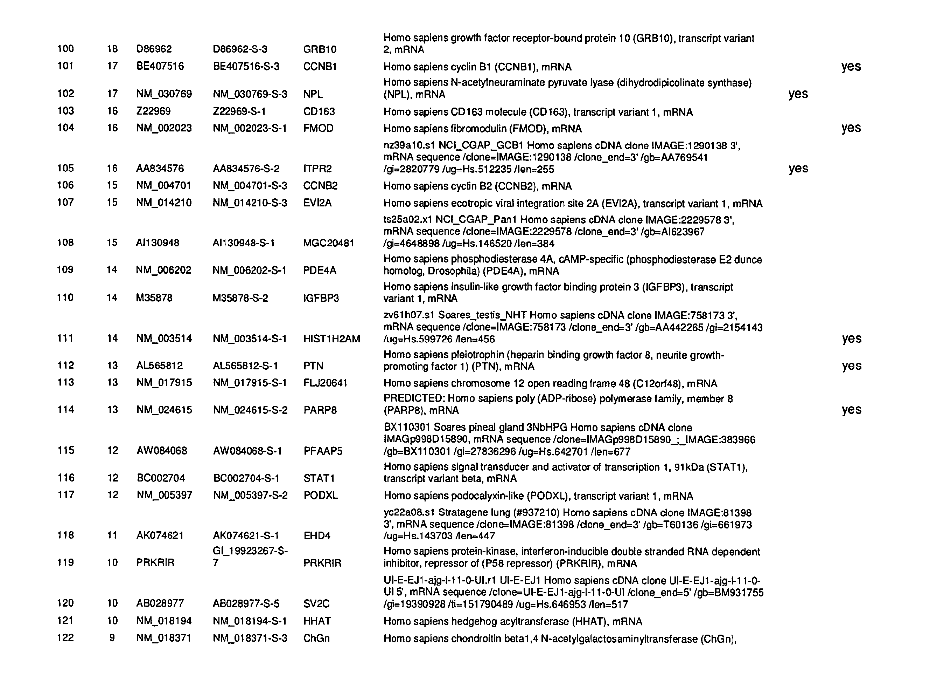

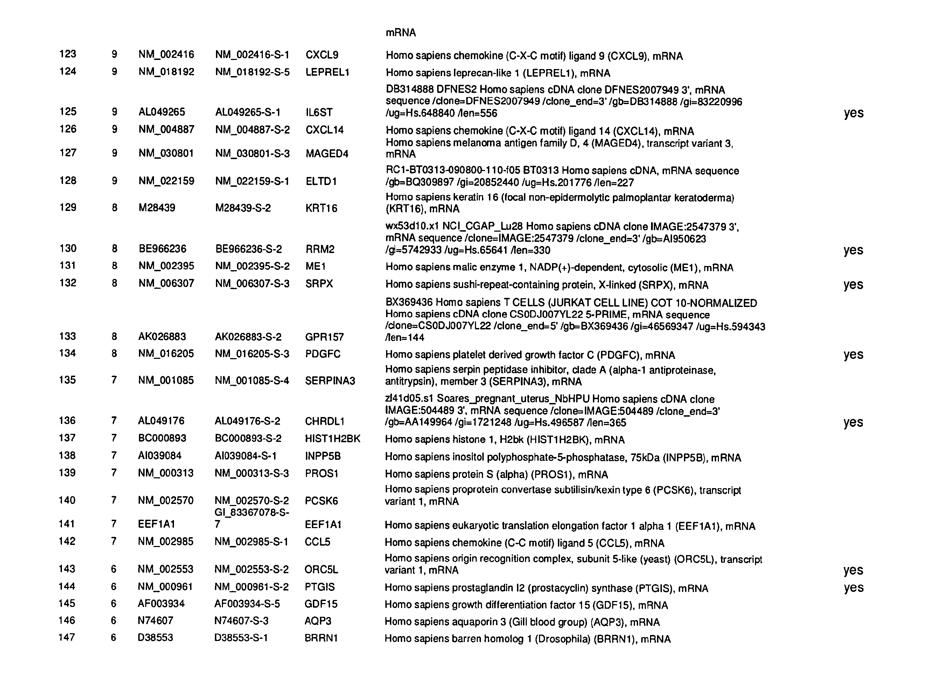

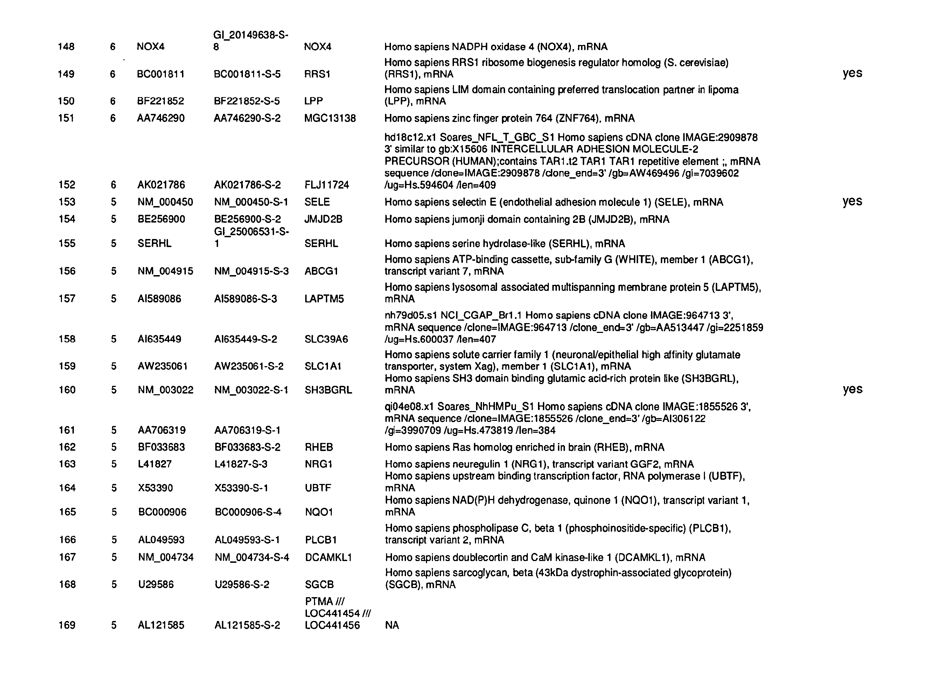

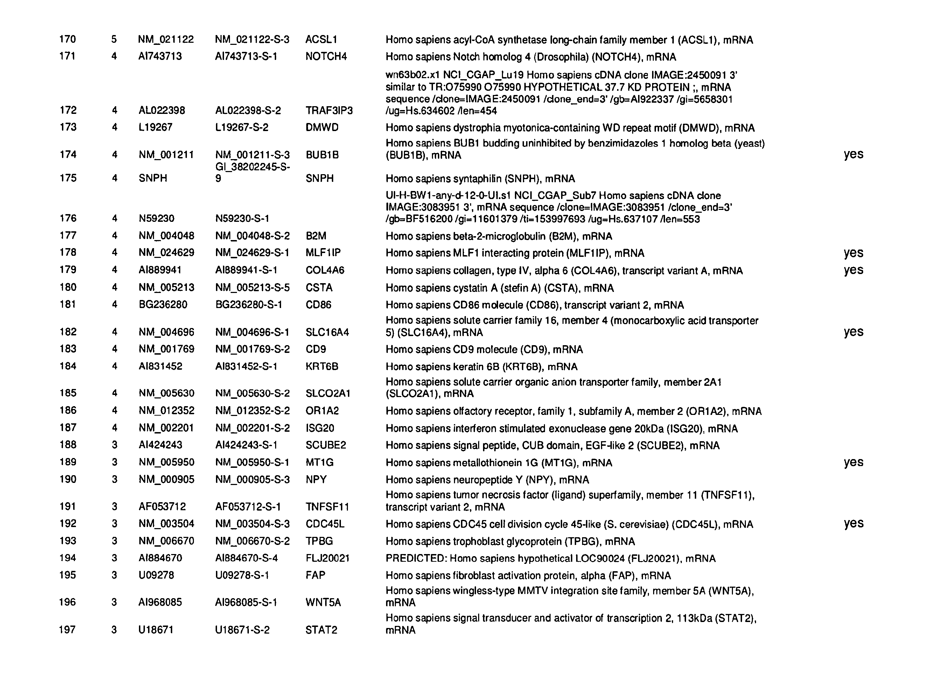

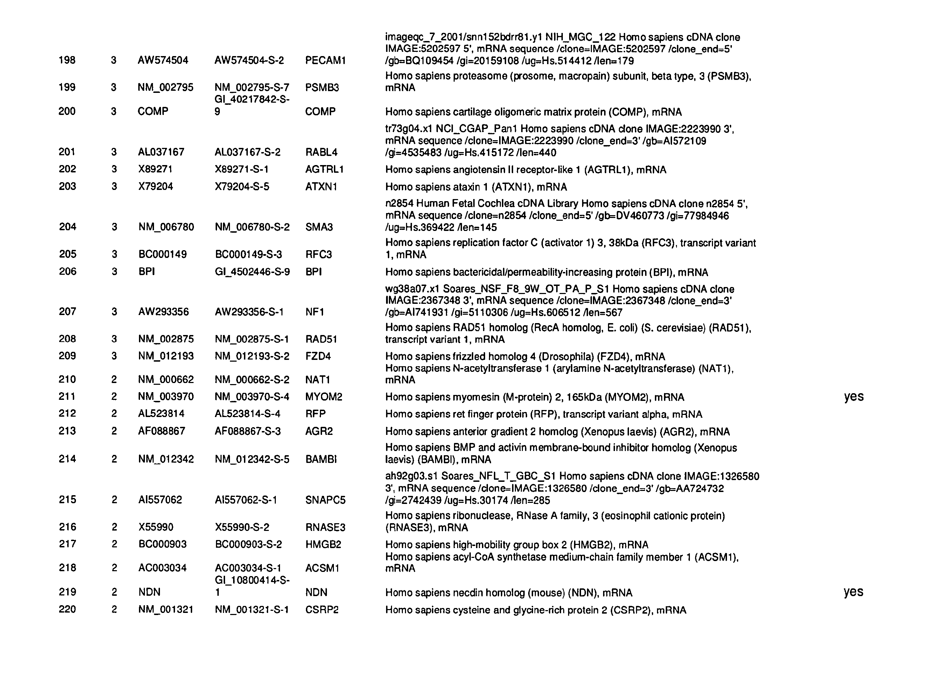

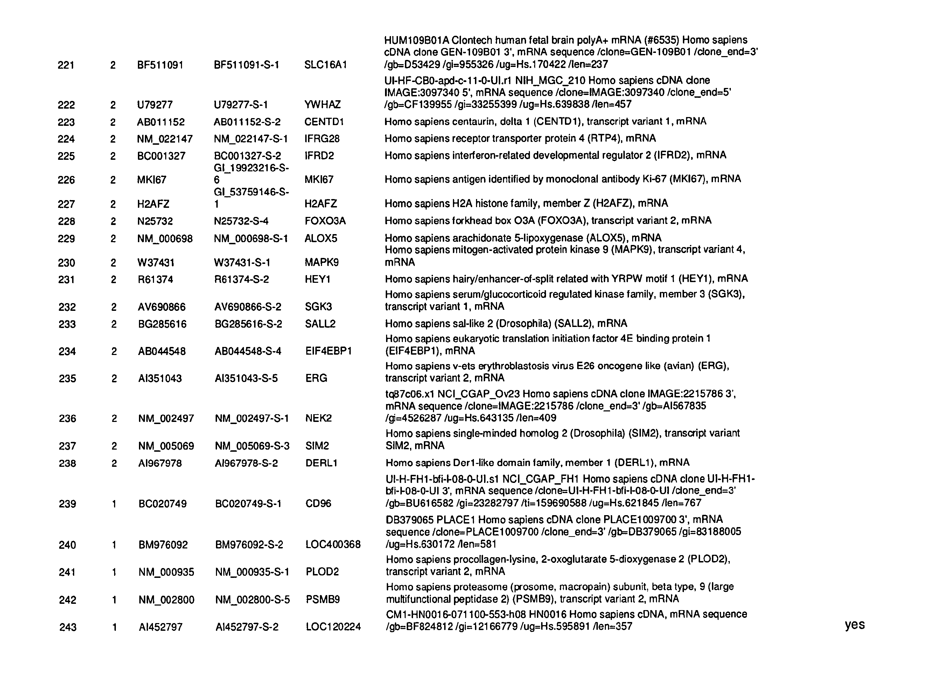

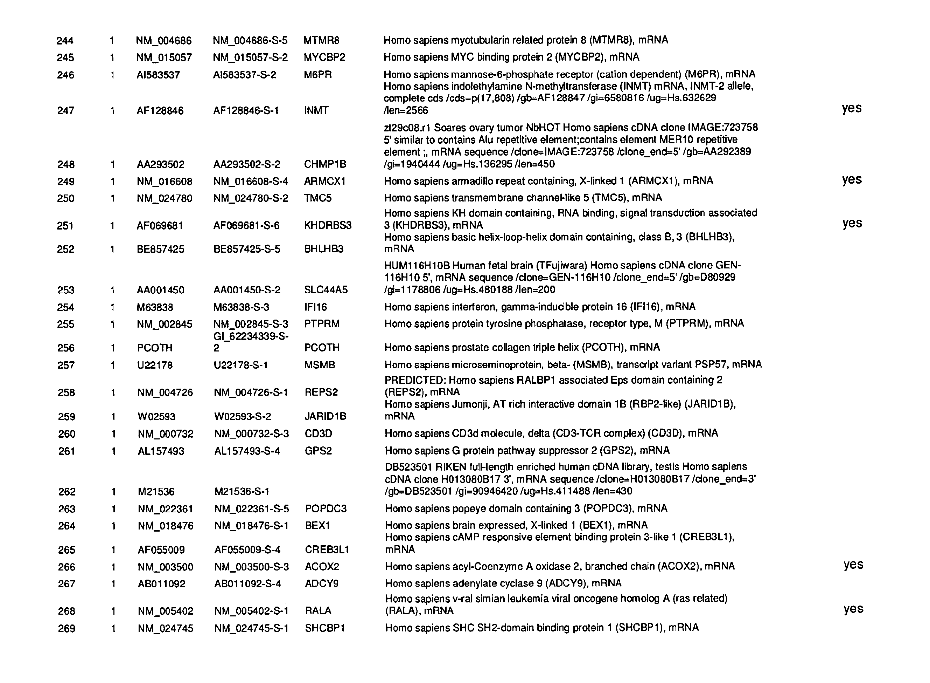

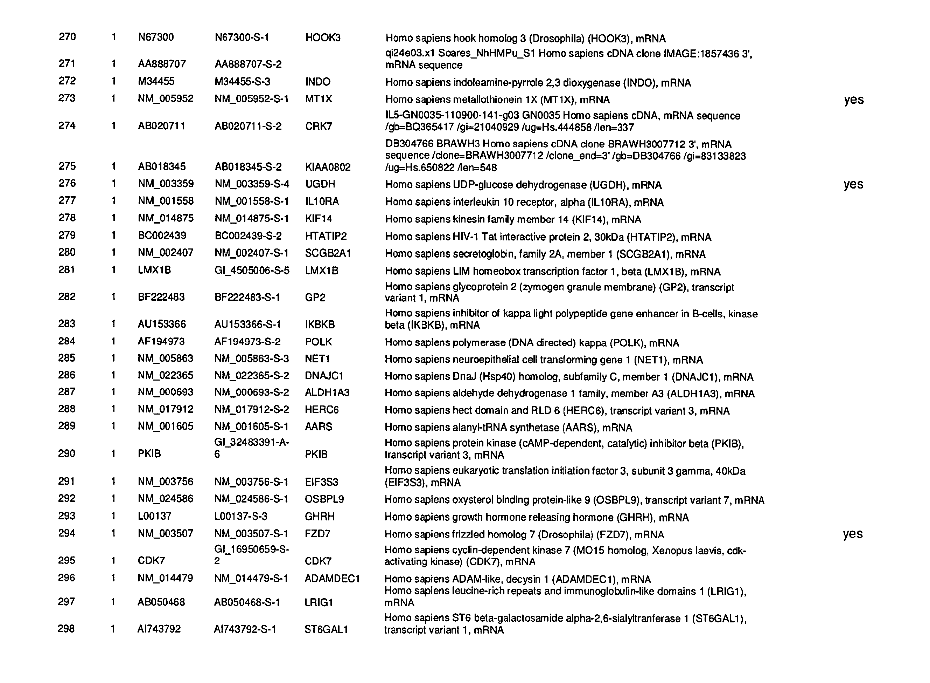

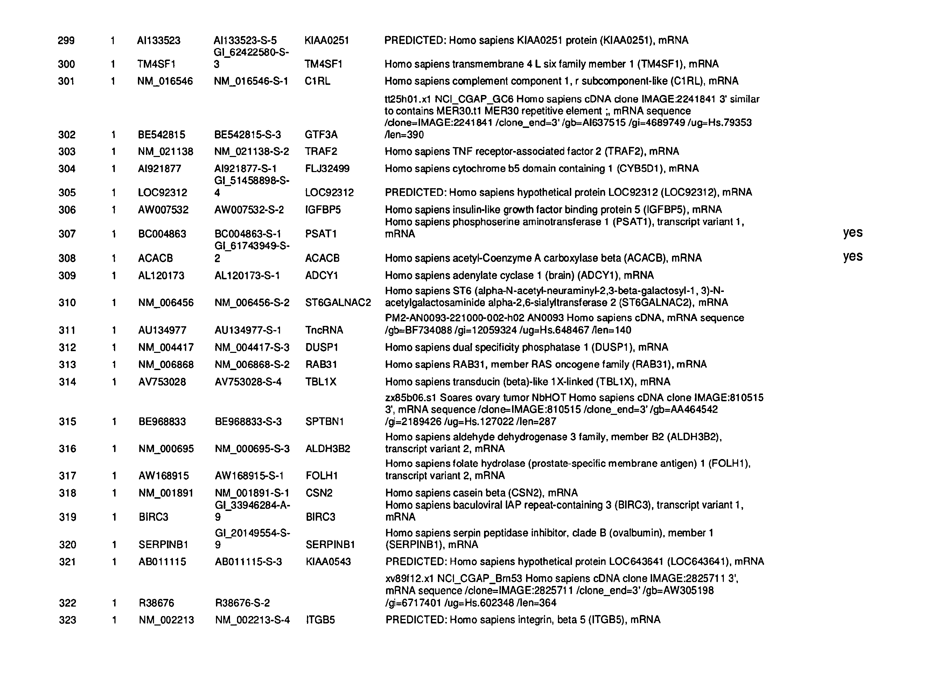

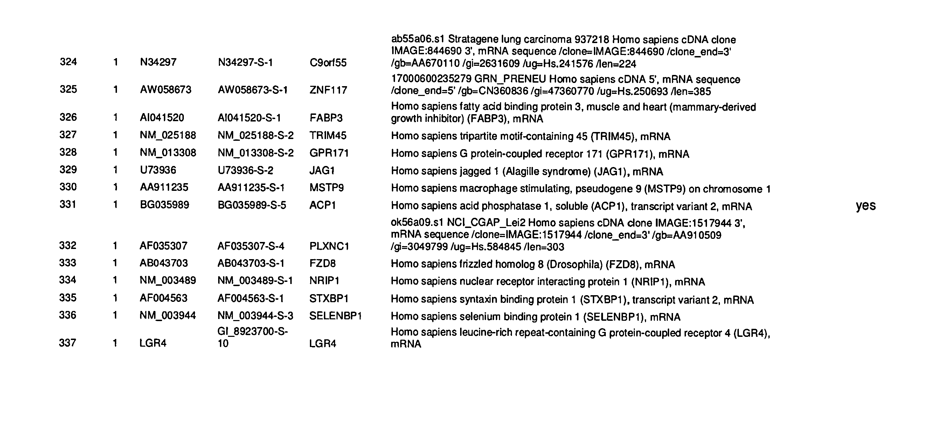

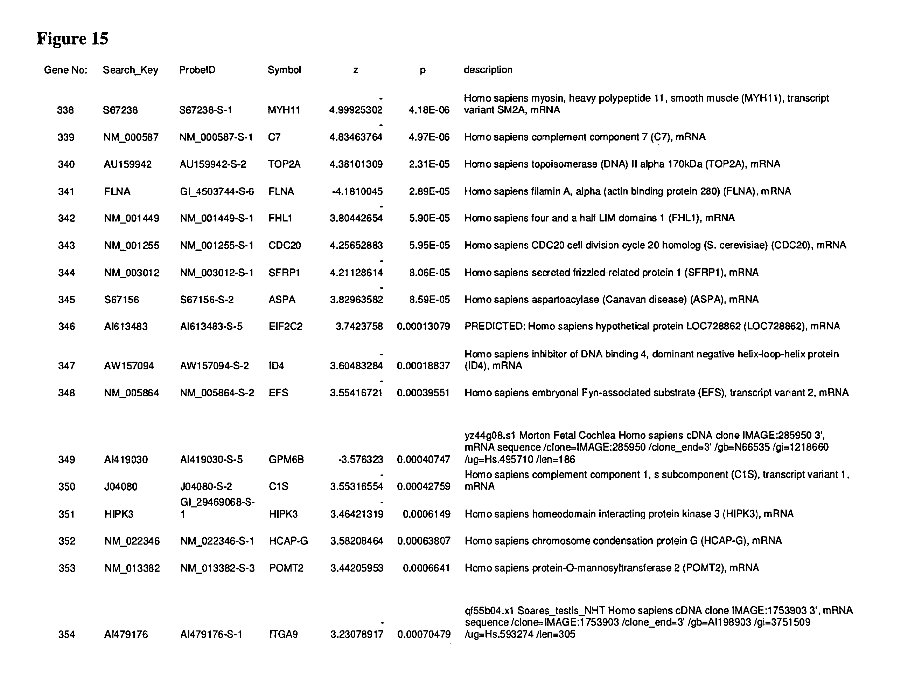

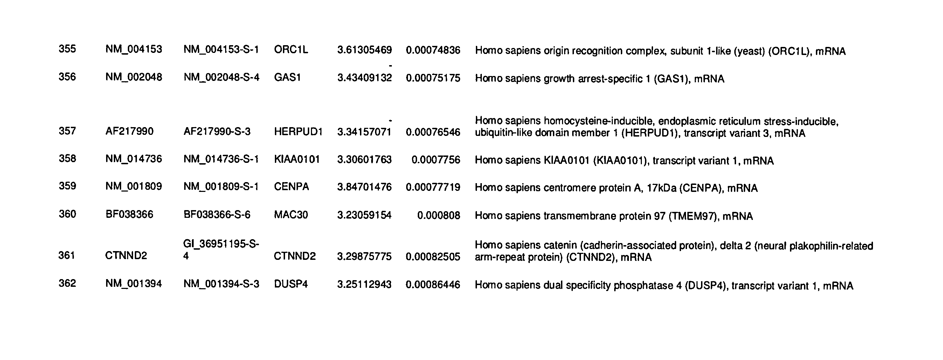

The present invention provides a method to determine the risk of prostate cancer recurrence in a subject, said method comprising
assaying a sample comprising prostate cancer cells from said subject for the expression levels of six or more genes selected from Gene Nos: 1 to 362 in Figures 14 and 15,
determining, as an aggregated value, a sum of the expression levels of said six or more genes, and
determining the risk of prostate cancer recurrence in said subject based upon said aggregated value,
wherein the expression levels are correlated with
a low risk of cancer recurrence, or
a high risk of cancer recurrence.

The present invention also provides a method to determine the risk of developing metastasis of prostate cancer in a subject, said method comprising
assaying a sample comprising prostate cancer cells from said subject for the expression levels of six or more genes selected from Gene Nos: 1 to 362 in Figures 14 and 15;
determining, as an aggregated value, a sum of the expression levels of said six or more genes, and
determining the risk of developing metastasis of prostate cancer in said subject based upon said aggregated value,
wherein the expression levels are correlated with low risk of developing metastasis of prostate cancer, or
a high risk of developing metastasis of prostate cancer.

### BRIEF SUMMARY OF THE DISCLOSURE

The disclosure, which encompasses the methods of the invention, also includes the identification and use of gene (or sequence) expression patterns (or profiles or "signatures") which are clinically relevant to prostate cancer. The gene expression profiles, whether embodied in nucleic acid expression, protein expression, or other expression formats, may be used to predict prostate cancer recurrence and/or survival of subjects afflicted with prostate cancer. In embodiments involving use of prostatectomy, the disclosure includes methods to predict the likelihood of prostate cancer recurrence, cancer metastasis, and/or recurrence of elevated PSA levels.

In one aspect, the disclosure includes methods to identify, and then use, gene (or sequence) expression profiles which provide prognostic information related to prostate cancer. The expression profiles correlate with (and so are able to discriminate between) patients with good or poor cancer recurrence, cancer metastasis, and/or survival outcomes. In some embodiments, the disclosure includes a method to identify expression profiles relevant to cancer recurrence and/or metastasis in prostate cancer afflicted subjects. In other embodiments, the disclosure includes a method to compare gene (or sequence) expression in a sample of prostate cancer cells from a patient to an identified expression profile to determine the likely outcome for the patient, such as after prostatectomy. These embodiments of the disclosure may be advantageously used to meet an important unmet diagnostic need for the ability to predict whether a patient will likely benefit from surgery to treat prostate cancer or whether a patient will be better off with another type of treatment or need adjuvant treatment in addition to surgery.

One non-limiting example of a patient that will likely benefit from surgery, such as a radical prostatectomy, is one predicted, by the expression profile of the patient's prostate cancer cells as disclosed herein, to be free of cancer recurrence and/or metastasis following the surgery. In a related manner, a non-limiting example of a patient that may be expected to not benefit from surgery is one predicted, by the expression profile of the patient's prostate cancer cells as disclosed herein, to develop cancer metastasis following the surgery. By way of example, the recurrence of prostate cancer may be at the same location while metastasis is frequently to a different location or tissue, such as to a lymph node or bone as non-limiting examples.

In further embodiments, a method to identify a patient, from a population of patients with prostate cancer cells, or following prostatectomy, as belonging to a subpopulation of patients with a better cancer recurrence and/or survival outcome (such as lower risk of recurrence or metastasis), relative to another subpopulation, or as belonging to a subpopulation with a poorer cancer recurrence and/or survival outcome (such as elevated risk of recurrence or metastasis), relative to another subpopulation. The method is thus able to distinguish patients into at least two subpopulations or subtypes.

The disclosure includes a non-subjective means for the identification of patients with prostate cancer, optionally following prostatectomy, as likely to have a better or poorer cancer recurrence, cancer metastasis, and/or survival outcome by assaying for an expression pattern disclosed herein. Thus where subjective interpretation (such as that based upon immunohistochemical staining and subjective analysis) may have been previously used to determine the prognosis and/or treatment of prostate cancer patients, or post-prostatectomy patients, this disclosure introduces objective expression patterns, which may used alone or in combination with other (subjective and/or objective) criteria to provide a more accurate assessment of patient outcomes, including survival and the recurrence or metastasis of cancer. The expression patterns of the disclosure thus include a means to determine prostate cancer, or post-prostatectomy, prognosis.

The expression patterns of the disclosure comprise more than one gene, or expressed sequence, capable of discriminating between prostate cancer, or post-prostatectomy, outcomes with significant accuracy. The expression level(s) of the gene(s), or expressed sequence(s) are identified as correlated with outcomes such that the expression level(s) is/are relevant to a determination of the preferred treatment protocols of a patient. Thus, the disclosure includes a method to determine the outcome of a subject afflicted with prostate cancer, optionally post-prostatectomy, by assaying a prostate cancer cell containing sample from said subject for expression levels that are correlated with patient outcomes as disclosed herein.

In another aspect, the disclosure includes a method to determine the risk of prostate cancer recurrence and/or metastasis in a subject. The method includes assaying a sample comprising prostate cancer cells from said subject for the expression levels of two or more genes selected from Gene Nos: 1 to 362 in Figures 14 and 15 herein, and comparing the expression level of each gene individually, or the aggregated expression levels *in toto,* with the mean or median expression level(s) thereof in a population of prostate cancer cells. This assessment may then be used to determine the risk of prostate cancer recurrence and/or metastasis in the subject from which the sample was obtained. The expression levels of the disclosed genes (or expressed sequences) are correlated with a low risk of cancer recurrence and/or metastasis, or a high risk of cancer recurrence and/or metastasis, based upon comparison to the mean or median level of expression in a population of prostate cancer cells. Put differently, the individual genes (or expressed sequences) disclosed herein are expressed at higher, or lower, levels (in comparison to the mean or median level of expression in a prostate cancer cell population) such that the deviation from the mean or median is correlated with an higher or lower risk as described herein. Each aggregation of deviations in two or more genes (or expressed sequences) is an expression pattern or profile of the disclosure.

In some embodiments, the assaying for expression levels may be performed by use of two or more nucleic acid probes selected from SEQ ID NOs: 1-362 as disclosed herein. These probes hybridize, under appropriate conditions, and detect expressed sequences as disclosed herein. In other embodiments, other probes which hybridize to the same expressed sequences as SEQ ID NOs: 1-362 may be used. These additional probes may be prepared or selected by analysis of the expressed sequences detected by SEQ ID NOs: 1-362. In other cases, additional probe sequence may be all or part of the sequences identified as Gene Nos: 1-362. In many embodiments, a probe may be used to detect a region of sequence amplified from an expressed sequence (or gene) as described herein.

The detection or determination of expression levels of two or more (gene) sequences may be referred to as detecting or determining an expression profile or signature. Expression patterns of the disclosure comprising two or more of the disclosed sequences are identified and used as described herein. For their identification, a large sampling of the gene expression levels in a sample containing prostate cancer cells is obtained through quantifying the expression levels of mRNA. In one embodiment, the disclosure includes detecting gene (or sequence) expression levels by analyzing global, or near global, gene expression from single cells or homogenous cell populations which have been dissected away from, or otherwise isolated or purified from, contaminating cells beyond that possible by a simple biopsy. Because the expression of numerous genes fluctuate between cells from different patients as well as between cells from the same patient sample, multiple data from expression of individual gene sequences are used as reference data to generate models which in turn permit the identification of individual genes and sequences, the expression of which are correlated with particular prostate cancer, or post-prostatectomy, outcomes.

The expression levels of various genes in these models are then analyzed to identify nucleic acid sequences, the expressions of which are positively, or negatively, correlated, with a prostate cancer, or post-prostatectomy, outcome. This disclosure includes nucleic acid sequences, which are a subset of expressed sequences in a cell, identified as correlating to outcomes as described herein. An expression pattern or profile of the disclosure includes a combination of these identified sequences (or genes). The use of multiple samples for identification of expressed sequences increases the confidence with which a gene is considered to correlate with a prostate cancer recurrence, metastasis, and/or survival outcome. Without sufficient confidence, it remains unpredictable whether a particular gene is actually correlated with an outcome and also unpredictable whether expression of a gene may be successfully used to identify the outcome for a prostate cancer, or post-prostatectomy, subject or patient. Once identified, two or more nucleic acid sequences corresponding to the disclosed genes (or expressed sequences) may be selected for assessment as an expression profile to be detected or assessed for its predictive properties.

A profile of expression levels that is highly correlated with one outcome relative to another may be used to assay a prostate cancer cell containing sample from a subject or patient to predict the outcome of the subject from whom the sample was obtained. Such an assay may be used as part of a method to determine the therapeutic treatment for said subject based upon the outcome identified.

The correlated genes may be used in pairs (with significant accuracy) or in or in higher numbers to increase the ability to accurately correlate a molecular expression phenotype with a prostate cancer, or post-prostatectomy, outcome. This correlation provides a molecular determination of prostate cancer recurrence, cancer metastasis, and/or survival outcomes as disclosed herein. Without being bound by theory, and offered to improve understanding of the instant disclosure, the disclosed molecular determination is more powerful than other molecular indicators related to prostate cancer, such as the determination of prostate serum antigen (PSA) levels. Additional uses of the correlated expression patterns and profiles are in the classification of cells and tissues; determination of diagnosis and/or prognosis; and determination and/or alteration of therapy.

The ability to discriminate is conferred by the identification of expression of the individual gene sequences as relevant and not by the form of the assay used to determine the actual level of expression. An assay may utilize any identifying feature of an identified individual gene as disclosed herein as long as the assay reflects, quantitatively or qualitatively, expression of the gene sequence in the "transcriptome" (the transcribed fraction of genes in a genome) or the "proteome" (the translated fraction of expressed genes in a genome). Identifying features include, but are not limited to, unique nucleic acid sequences used to encode (DNA), or express (RNA), said gene or epitopes specific to, or activities of, a protein encoded by said gene. All that is required is the identity of the gene sequence(s) necessary to discriminate between prostate cancer, or post-prostatectomy, outcomes and an appropriate cell containing sample for use in an expression assay.

Similarly, the nature of the cell containing sample is not limiting, as fresh tissue, freshly frozen tissue, and fixed tissue, such as formalin-fixed paraffin-embedded (FFPE) tissues, may be used in the disclosed methods. In some embodiments, the sample may be that of a needle (core) biopsy or other biopsy.

For detecting an identified expression pattern or profile, the disclosure includes detecting gene (or sequence) expression patterns by gathering global, or near global, gene expression from single cells or homogenous cell populations which have been dissected away from, or otherwise isolated or purified from, contaminating cells beyond that possible by a simple biopsy. The expression levels of the genes (sequences) in the pattern or profile are then detected or otherwise measured. In other embodiments, a method may only detect or measure the expression levels of the genes (sequences) in the profile without assessment or other determination of the expression of other genes or sequences.

In an additional aspect, the analysis of expression levels may be performed in combination with, or in place of, other assessments or indicators of prostate cancer. In some embodiments, the analysis is made in combination with a method of determining the grade of prostate cancer in a sample comprising prostate cancer cells from a subject. In other embodiments, the combination is with a method of determining the stage of prostate cancer in the sample. A third possibility is combination with detecting or determining PSA levels in the subject, optionally before a procedure used to isolate the prostate cancer cells. Of course a combination with any one, two, or all three of these representative examples is possible. Whenever more than one type of assessment is used, the result is a multivariate analysis. The disclosure expressly includes all possible combinations of assessments described herein as multivariate embodiments.

Generally, any accepted method of assessing prostate cancer grade and/or stage as known to the skilled person may be used. In some cases, the method of determining prostate cancer grade comprises determination of a Gleason Score (or Gleason Grade). In other cases, the method of determining prostate cancer stage comprises a determination according to the American Joint Committee on Cancer (AJCC) tumor staging system for assessing prostate cancer stage. And as described herein, the analysis of gene (sequence) expression levels may be performed in place of either the Gleason Score or the AJCC tumor stage determination.

In cases of PSA levels, its assessment may be conducted before a prostatectomy which is used to provide a sample comprising prostate cancer cells for use in any method described herein..

In a further aspect, the disclosure includes physical and methodological means for detecting the expression of genes (or sequences) disclosed herein. These means may be directed to assaying one or more aspect of the DNA template(s) underlying the expression of the gene (or sequence), of the RNA used as an intermediate to express the gene (or sequence), or of the proteinaceous product expressed by the gene (or sequence).

One advantage provided by the disclosure is that contaminating, non-prostate cancer cells (such as infiltrating lymphocytes or other immune system cells) may be removed to reduce their effect on measurements of the expression patterns or profiles disclosed herein to predict the cancer recurrence and/or survival outcomes of patients. Such contamination is present where a biopsy containing many cell types is used to generate gene expression profiles.

While the present disclosure is described mainly in the context of human prostate cancer, it may be practiced in the context of prostate cancer of any animal known to be potentially afflicted by prostate cancer. Non-limiting examples of animals for the application of the present disclosure are mammals, particularly those important to agricultural applications (such as, but not limited to, cattle, sheep, horses, and other "farm animals"), animal models of prostate cancer, and animals for human companionship (such as, but not limited to, dogs and cats).

### BRIEF DESCRIPTION OF THE FIGURES

Figure 1 illustrates a representative data analysis scheme, based on human patient samples as described in Example 1 and Table 1 herein.
Figure 2 shows the result of 500 gene selection iterations of the Random Forests™ algorithm as described herein.
Figure 3A shows a representative identification of prostate cancer on a slide containing a radical prostatectomy specimen (FFPE tissue) with an area of cancerous tissue identified. Figure 3B shows the excised (macro-dissected) prostate cancer material after removal of non-cancerous tissue.
Figure 4 shows correlation between the gene risk signature and Gleason Grade or score (<=6, 7 or >=8) of samples from the training and test sets.
Figure 5 shows correlation between the gene risk signature and AJCC pathologic tumor stage (II or III) of samples from the training and test sets.
Figure 6 shows correlation between the gene risk signature and pre-operative PSA (prostate serum antigen) value of samples from the training and test sets.
Figure 7 shows results from Kaplan-Meier analysis for freedom from PSA failure (increase of PSA values) after surgical intervention to treat prostate cancer. The results show segregation into a "low" risk subpopulation with better cancer recurrence and probability of survival outcomes (lower incidence of recurrence and death due to metastasis) and a "high" risk subpopulation with poorer outcomes (higher incidence of recurrence and metastasis).

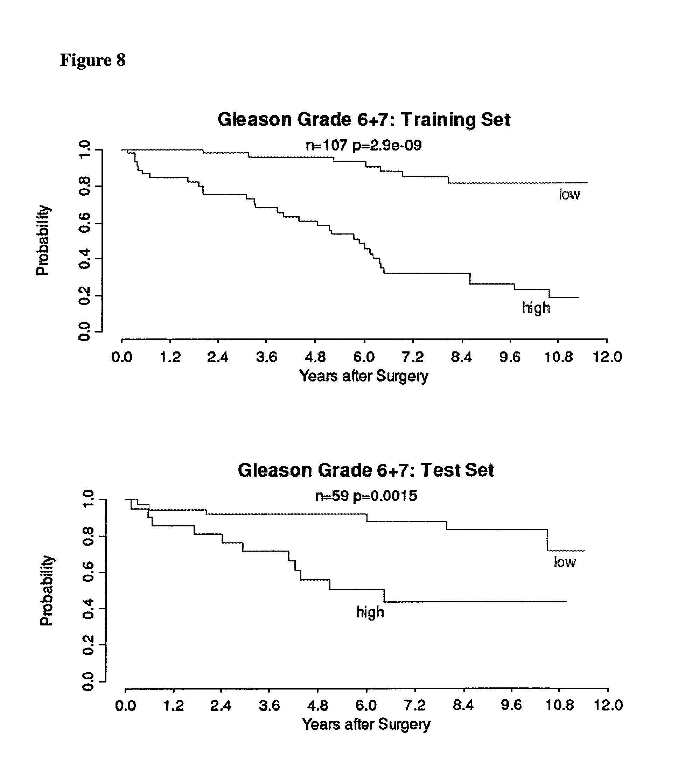

Figure 8 shows results from Kaplan-Meier analysis for freedom from PSA failure after surgical intervention to treat prostate cancer in cells samples with Gleason Grade or scores of <=6 and 7. The results show segregation into a "low" risk subpopulation with better cancer recurrence and probability of survival outcomes (lower incidence of recurrence and death due to metastasis) and a "high" risk subpopulation with poorer cancer recurrence outcomes (higher incidence of recurrence and metastasis). Similar results are observed if the samples are separated into those with Gleason Grade or score of <=6, score of 7, or score of >=8.

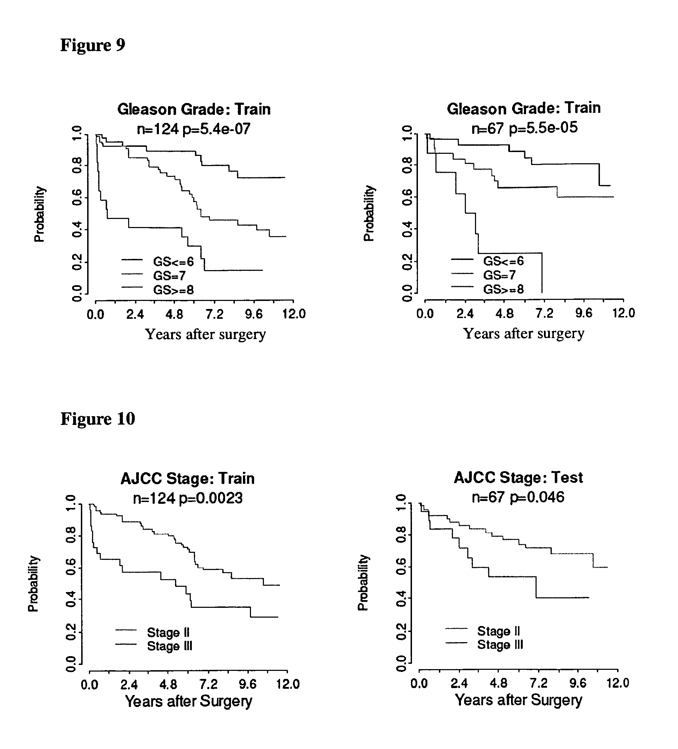

Figure 9 shows survival curves based on Gleason Grade alone.
Figure 10 shows survival curves based on AJCC Stage alone.

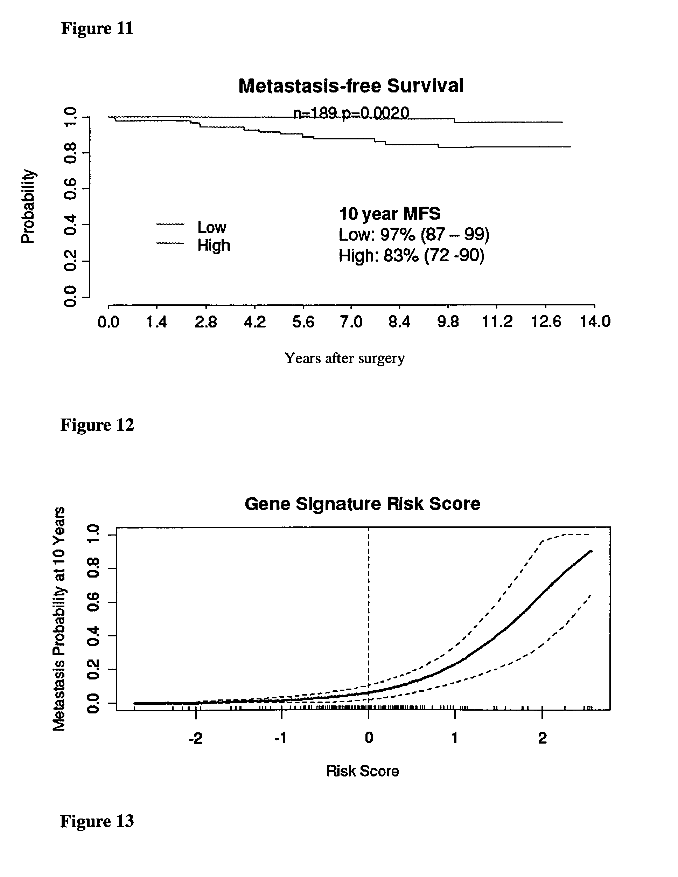

Figure 11 shows the results of using a disclosed Risk Score (based on 62 representative genes) to segregate 189 patients into a "low" risk subpopulation with better probability of survival outcomes (lower incidence of death due to metastasis) and a "high" risk subpopulation with poorer outcomes (higher incidence of metastasis) over 10 years.
Figure 12 shows a plot of the Figure 11 Risk Score versus the probability of metastasis within 10 years. As indicated by the graph, a Score over 0 in prostate cancer cells of a subject indicates an increasing risk of metastasis occurring within 10 years following surgical intervention.

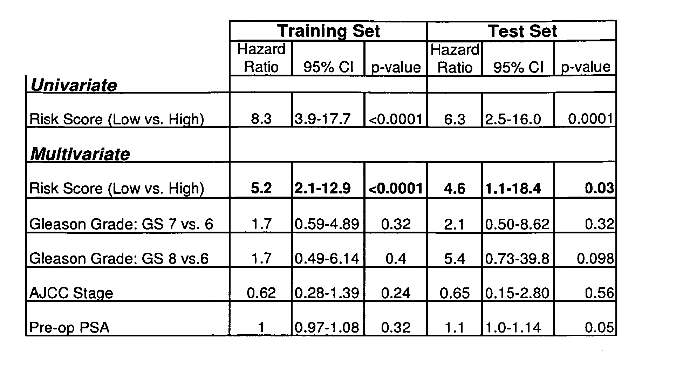

Figure 13 summarizes and compares the use of univariate and multivariate analysis for PSA failure. The indicated AJCC Stage is II versus III. CI refers to confidence interval.
Figure 14 lists the annotations and identifying information for 337 genes for use as described herein. The Gene No. may be used to cross reference each gene with the corresponding SEQ ID NO of the probe and its performance information in Table 1 below. The frequency with which the identified molecules occur among 500 sets generated (as described in Example 1) is indicated in the "freq" column. The remaining columns contain identifying information for each gene, such as gene accession number ("Search Key" column), accession number for the corresponding probe sequence in Table 1 ("ProbeID" column), gene name symbol ("Symbol" column), and a brief description (last column).
Figure 15 lists the annotations and identifying information for an additional 25 genes for use as described herein. The Gene No. and performance information (z and p values) may be used to cross reference each gene with the corresponding SEQ ID NO of the probe and its performance information in Table 3 below. Identifying information for each gene includes gene accession number ("Search Key" column), accession number for the corresponding probe sequence in Table 3 ("ProbeID" column), gene name symbol ("Symbol" column), and a brief description (last column).

### DETAILED DESCRIPTION OF MODES OF PRACTICING THE DISCLOSURE

### Definitions of terms

A gene expression "pattern" or "profile" or "signature" refers to the relative expression of two or more genes (expressed sequences) between two or more prostate cancer, or post-prostatectomy, cancer recurrence, metastasis, and/or survival outcomes which expression is correlated with being able to distinguish between the outcomes.

A "gene" or "expressed sequence" is a polynucleotide that encodes, and expresses in a detectable manner, a discrete product, whether RNA or proteinaceous in nature. It is appreciated that more than one polynucleotide may be capable of encoding a discrete product. The term includes alleles and polymorphisms of a gene or expressed sequence that encodes the same product, or a functionally associated (including gain, loss, or modulation of function) analog thereof, based upon chromosomal location and ability to recombine during normal mitosis.

The terms "correlate" or "correlation" or equivalents thereof refer to an association between expression of two or more genes and a physiologic state of a prostate cell to the exclusion of one or more other states as identified by use of the methods as described herein. A gene may be expressed at higher or lower levels and still be correlated with one or more prostate cancer, or post-prostatectomy, state or outcome. One way to express correlation is with a z value as disclosed herein for the expression of various genes (or expressed sequences). The z value may be viewed as indicating the strength, or weight, of the association between the expression level of a gene (or expressed sequence) and a particular outcome, such as cancer recurrence, cancer metastasis, and/or survival over time. The value may have a positive or negative (+/-) sign which arbitrarily, but consistently, indicates over or under expression, respectively. Because the signs are arbitrary, their assignment can be readily (but consistently) reversed without deleterious effect.

In some embodiments, the strength (or weight) is multiplied by the expression level of a given gene (or expressed sequence), after normalization thereof (such as relative to expression of a reference gene that is expressed at relatively constant levels) to provide a value or score for the expression of that gene (or sequence). In many cases, the expression data is normalized, median-centered, and log-transformed as known to the skilled person prior to further use, such as in clustering and discriminant analysis. Where more than one expression level is used (as in the case of a gene expression profile or pattern), the values or scores may be summed and then analyzed as an aggregate value for assessing the correlation or conducting classification based on the correlation.

A "polynucleotide" is a polymeric form of nucleotides of any length, either ribonucleotides or deoxyribonucleotides. This term refers only to the primary structure of the molecule. Thus, this term includes double- and single-stranded DNA and RNA. It also includes known types of modifications including labels known in the art, methylation, "caps", substitution of one or more of the naturally occurring nucleotides with an analog, and internucleotide modifications such as uncharged linkages (e.g., phosphorothioates, phosphorodithioates, etc.), as well as unmodified forms of the polynucleotide.

The term "amplify" is used in the broad sense to mean creating an amplification product can be made enzymatically with DNA or RNA polymerases. "Amplification," as used herein, generally refers to the process of producing multiple copies of a desired those of a sample. "Multiple copies" mean at least 2 copies. A "copy" does not necessarily mean perfect sequence complementarity or identity to the template sequence.

The term "corresponding" can refers to, where appropriate, a nucleic acid molecule as sharing a substantial amount of sequence identity with another nucleic acid molecule. Substantial amount means at least 95%, usually at least 98% and more usually at least 99%, and sequence identity is determined using the BLAST algorithm, as described in Altschul et al. (1990), J. Mol. Biol. 215:403-410 (using the published default setting, i.e. parameters w=4, t=17). Methods for amplifying mRNA are generally known in the art, and include reverse transcription PCR (RT-PCR) and those described in U.S. Patent Application 10/062,857 (filed on October 25, 2001), as well as U.S. Provisional Patent Applications 60/298,847 (filed June 15, 2001) and 60/257,801 (filed December 22, 2000).

Another method which may be used is quantitative PCR (or Q-PCR). Alternatively, RNA may be directly labeled as the corresponding cDNA by methods known in the art.

A "microarray" is a linear or two-dimensional array of discrete regions, each having a defined area, formed on the surface of a solid support such as, but not limited to, glass, plastic, or synthetic membrane. The density of the discrete regions on a microarray is determined by the total numbers of immobilized polynucleotides to be detected on the surface of a single solid phase support, such as at least about 50/cm², at least about 100/cm², at least about 500/cm², but below about 1,000/cm² in some embodiments. The arrays may contain less than about 500, about 1000, about 1500, about 2000, about 2500, or about 3000 immobilized polynucleotides in total. As used herein, a DNA microarray is an array of oligonucleotides or polynucleotides placed on a chip or other surfaces used to hybridize to amplified or cloned polynucleotides from a sample. Because the position of each particular group of polynucleotides in the array is known, the identities of a sample polynucleotides can be determined based on their binding to a particular position in the microarray.

Because the disclosure relies upon the identification of genes (or expressed sequences) that are over- or under-expressed, one embodiment of the disclosure involves determining expression by hybridization of mRNA, or an amplified or cloned version thereof (such as DNA or cDNA), of a sample cell to a polynucleotide that is unique to a particular gene sequence. Polynucleotides of this type may contain at least about 20, at least about 22, at least about 24, at least about 26, at least about 28, at least about 30, or at least about 32 consecutive basepairs of a gene sequence that is not found in other gene sequences. The term "about" as used in the previous sentence refers to an increase or decrease of 1 from the stated numerical value. Other embodiments may use polynucleotides of at least or about 50, at least or about 100, at least about or 150, at least or about 200, at least or about 250, at least or about 300, at least or about 350, or at least or about 400 basepairs of a gene sequence that is not found in other gene sequences. The term "about" as used in the preceding sentence refers to an increase or decrease of 10% from the stated numerical value. Such polynucleotides may also be referred to as polynucleotide probes that are capable of hybridizing to sequences of the genes, or unique portions thereof, described herein. In many cases, the hybridization conditions are stringent conditions of about 30% v/v to about 50% formamide and from about 0.01M to about 0.15M salt for hybridization and from about 0.01M to about 0.15M salt for wash conditions at about 55 to about 65°C or higher, or conditions equivalent thereto.

In other embodiments, other probes which hybridize to the same expressed sequences as SEQ ID NOs: 1-362 may be used. These additional probes may be prepared or selected by analysis of the expressed sequences detected by SEQ ID NOs: 1-362. Such additional polynucleotide probes for use in the disclosure may have about or 95%, about or 96%, about or 97%, about or 98%, or about or 99% identity with the gene sequences to be used. Identity is determined using the BLAST algorithm, as described above. These additional probes may also be described on the basis of the ability to hybridize to expressed genes and sequences of the disclosure under stringent conditions as described above or conditions equivalent thereto.

In many cases, the sequences are those of mRNA encoded by the genes, the corresponding cDNA to such mRNAs, and/or amplified versions of such sequences. In some embodiments of the disclosure, the polynucleotide probes are immobilized on an array, other devices, or in individual spots that localize the probes. In many embodiments, the probes are directed to a region of a gene (or expressed sequence) that is, or contains, a part of a 3' untranslated region. In some cases, the region is within about 100, 200, 300, 400, 500, 600, 700, 800, or 900 or more nucleotides of the polyadenylation signal or polyadenylated tail in an mRNA.

Alternatively, and in another embodiment of the disclosure, gene expression may be determined by analysis of expressed protein in a cell sample of interest by use of one or more antibodies specific for one or more epitopes of individual gene products (proteins) in said cell sample. Such antibodies are may be labeled to permit their easy detection after binding to the gene product.

The term "label" refers to a composition capable of producing a detectable signal indicative of the presence of the labeled molecule. Suitable labels include radioisotopes, nucleotide chromophores, enzymes, substrates, fluorescent molecules, chemiluminescent moieties, magnetic particles, bioluminescent moieties, and the like. As such, a label is any composition detectable by spectroscopic, photochemical, biochemical, immunochemical, electrical, optical or chemical means.

The term "support" refers to conventional supports such as beads, particles, dipsticks, fibers, filters, membranes and silane or silicate supports such as glass slides.

As used herein, a "prostate tissue sample" or "prostate cancer cell sample" refers to a sample of prostate tissue isolated from an individual, such as one afflicted with prostate cancer. The sample may be from material removed via a prostatectomy, such as a radical prostatectomy. Alternatively, they are obtained by other means, such as needle (core) biopsy or other biopsy techniques, like laterally directed biopsies, the conventional sextant biopsy approach, different combinations of sextant and lateral biopsies as extended techniques, transrectal ultrasound guided prostate biopsy, and others as known to the skilled person. Such samples are primary isolates (in contrast to cultured cells) and may be collected by any suitable means recognized in the art. In some embodiments, the "sample" may be collected by an invasive method, including, but not limited to, surgical biopsy. A sample may contain prostate tumor cells which are isolated by known methods or other appropriate methods as deemed desirable by the skilled practitioner. Isolation methods include, but are not limited to, microdissection, laser capture microdissection (LCM), or laser microdissection (LMD) before use in accordance with the disclosure.

"Expression" and "gene expression" include transcription and/or translation of nucleic acid material.

As used herein, the term "comprising" and its cognates are used in their inclusive sense; that is, equivalent to the term "including" and its corresponding cognates.

Conditions that "allow" an event to occur or conditions that are "suitable" for an event to occur, such as hybridization, strand extension, and the like, or "suitable" conditions are conditions that do not prevent such events from occurring. Thus, these conditions permit, enhance, facilitate, and/or are conducive to the event. Such conditions, known in the art and described herein, depend upon, for example, the nature of the nucleotide sequence, temperature, and buffer conditions. These conditions also depend on what event is desired, such as hybridization, cleavage, strand extension or transcription.

Sequence "mutation," as used herein, refers to any sequence alteration in the sequence of a gene disclosed herein interest in comparison to a reference sequence. A sequence mutation includes single nucleotide changes, or alterations of more than one nucleotide in a sequence, due to mechanisms such as substitution, deletion or insertion. Single nucleotide polymorphism (SNP) is also a sequence mutation as used herein. Because the present disclosure is based on the level of gene (or sequence) expression, mutations in non-coding, but regulatory, regions of genes as disclosed herein may also be assayed in the practice of the disclosure.

"Detection" includes any means of detecting, including direct and indirect detection of gene expression and changes therein. For example, "detectably less" products may be observed directly or indirectly, and the term indicates any reduction (including the absence of detectable signal). Similarly, "detectably more" product means any increase, whether observed directly or indirectly.

Prostatectomy refers to the removal of prostate tissue by a skilled clinician, such as a surgeon. Non-limiting examples include radical prostatectomy; open (traditional) prostatectomy (involving an incision through the perineum); laparoscopic prostatectomy; and robotic (nerve sparing) prostatectomy.

Gleason score refers to the grading of a sample of prostate cancer by a trained pathologist according to the Gleason system, which assigns a Gleason score using numbers from 1 to 5 based upon similarities in the cells of a sample of prostate tissue to cancerous tissue or normal prostate tissue. Tissue that looks much like normal prostate tissue is given a score or grade of 1 while a tissue that lacks normal features and the cells seem to be spread haphazardly through the prostate is given a score or grade of 5. Scores, or grades of 2 through 4, inclusive, have features in between these possibilities. But because prostate cancers may have areas with different scores or grades, separate scores or grades are given to the two areas that make up most of the tissue. The two scores or grades are added to yield a Gleason score (or Gleason sum) between 2 and 10.

Unless defined otherwise all technical and scientific terms used herein have the same meaning as commonly understood to one of ordinary skill in the art to which this disclosure belongs.

### General

The disclosure is based in part upon the discovery of gene (or sequence) expression-based predictors of prostate serum antigen (PSA) failure by using separate training and test sets of prostate cancer samples from archived FFPE tissues from 1993-1995. The samples had related long-term follow up clinical data from the patients, including those with no evidence of disease (NED) for at least 10 years post-surgery and those that developed a subsequent recurrence (such as distant metastasis), from whom the samples were obtained. 62, 92, 337 and 362 genes (or expressed sequences), from a starting set of about 1536 genes with a high degree of dynamic expression (or variation in expression levels) within different cancer types, were identified via RNA-based expression profiling of the samples. The expression levels of these genes correlate with PSA failure and clinical outcomes as described herein and therefore may be used as predictors as described herein. The identified genes have been reported to participate in cellular functions including cell cycle, blood coagulation and wound healing, transcription regulation, and apoptosis.

The predictive capability of the disclosed gene expression pattern (profile) may be correlated to Gleason score and AJCC tumor stage. It is also consistent with pre-operative PSA levels. The profile also has the ability to stratify samples with Gleason scores of <=6 and 7 into low and high risk of PSA failure. In multivariate analysis with known prognostic factors, the profile was the only predictor that remains statistically significant (p < 0.05). Therefore, detection of the profile at biopsy, or in tissue removed during prostatectomy, allows the distinguishing of indolent from aggressive cancers.

The disclosure includes the identification and use of gene expression patterns (or profiles or "signatures") which discriminate between (or are correlated with) prostate cancer survival and recurrence outcomes in a subject. Such patterns may be determined by the methods of the disclosure by use of a number of reference cell or tissue samples, such as those reviewed by a pathologist of ordinary skill in the pathology of prostate cancer, which reflect prostate cancer cells as opposed to normal or other non-cancerous cells. The outcomes experienced by the subjects from whom the samples may be correlated with expression data to identify patterns that correlate with the outcomes. Because the overall gene expression profile differs from person to person, cancer to cancer, and cancer cell to cancer cell, correlations between certain cells and genes expressed or underexpressed may be made as disclosed herein to identify genes that are capable of discriminating between prostate cancer outcomes.

### Identification and description of genes and sequences

The disclosure may be practiced with any two or more of the disclosed genes (expressed sequences) found to be differentially expressed with respect to prostate cancer, or post-prostatectomy, outcomes, such as post-surgical PSA rise of > 0.2 ng ("PSA failure"). The identification was made by using expression profiles of various homogenous prostate cancer cell populations. The expression level of each gene of the expression profile may be correlated with a particular outcome. Alternatively, the expression levels of two or more genes (expressed sequences) may be clustered (combined) and used based on correlations with particular outcomes. Non-limiting examples of outcomes include but not limited to, distant metastasis, such as to a lymph node or bone, or need for follow-up chemotherapy or radiation therapy or cryotherapy. In additional embodiments, the expression profile may be used to stratify subjects or patients into a differential prognosis of "watchful waiting" versus "need of further treatment" where the latter may include neoadjuvant or adjuvant therapy beyond surgery to remove the prostate cancer/tumor. The disclosed methods may thus be used as part of prostate cancer management.

Genes with significant correlations to prostate cancer (or post-prostatectomy) survival, metastasis, or recurrence outcomes are used to discriminate between outcomes. Alternatively, genes with significant correlations may be used in combination with genes with lower correlations to form an expression pattern or profile of the disclosure without significant loss of ability to discriminate between outcomes. Such combinations (or expression patterns) may be selected and tested by any appropriate means recognized in the art, including, but not limited to, cluster analysis, supported vector machines, neural networks or other algorithm known in the art. The patterns or profiles are capable of predicting the classification of an unknown sample based upon the expression of the genes used for discrimination in the models. "Leave one out" cross-validation may be used to test the performance of various combinations and to help identify weights (expressed genes or sequences) that are less informative or detrimental to the predictive ability of a combination. Cross-validation may also be used to identify genes or sequences, the expression of which enhance the predictive ability of the models.

The disclosed genes (sequences) expressed in correlation with particular prostate cancer, or post-prostatectomy, outcomes provide the ability to focus gene expression analysis to only those genes that contribute to the ability to stratify a subject among different outcomes. The expression of other genes in a prostate cancer cell would be relatively unable to provide information concerning, and thus assist in the differential prognosis (or discrimination) of, a prostate cancer outcome.

As will be appreciated by those skilled in the art, the combinations (expression patterns or profiles) are highly useful even when based on a small set of reference gene expression data and can become increasingly accurate with the inclusion of more reference data although the incremental increase in accuracy will likely diminish with each additional datum. The preparation of additional reference gene expression data using genes identified and disclosed herein for discriminating between different outcomes in prostate cancer, or post-prostatectomy, is routine and may be readily performed by the skilled artisan to permit the generation of models as described above to predict the status of an unknown sample based upon the expression levels of those genes.

The disclosure includes the expressed sequences in the following Table 1 and expressed genes in Figure 14.

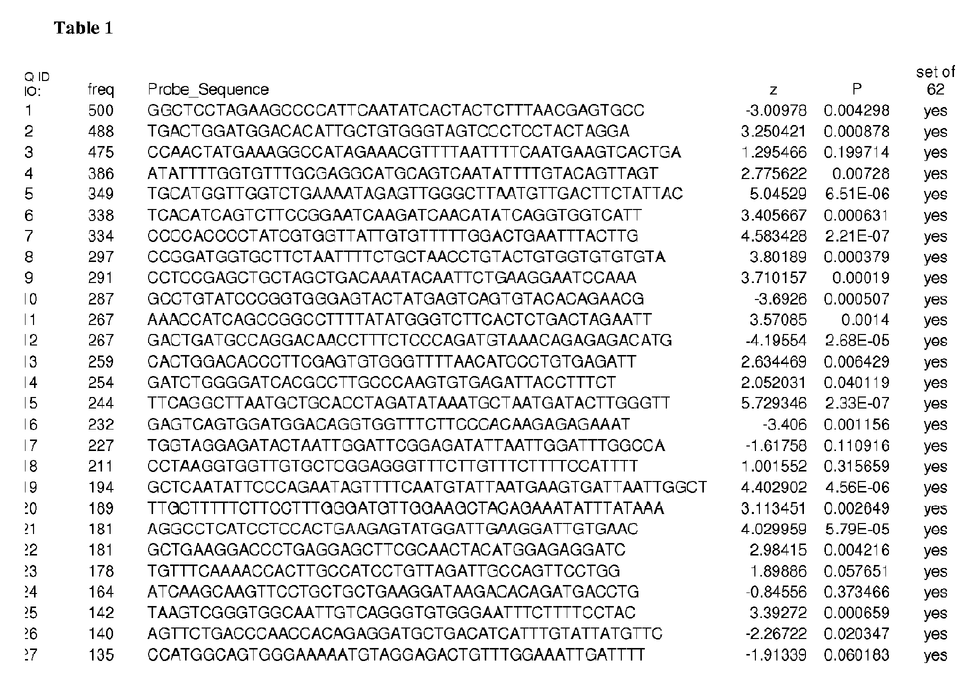

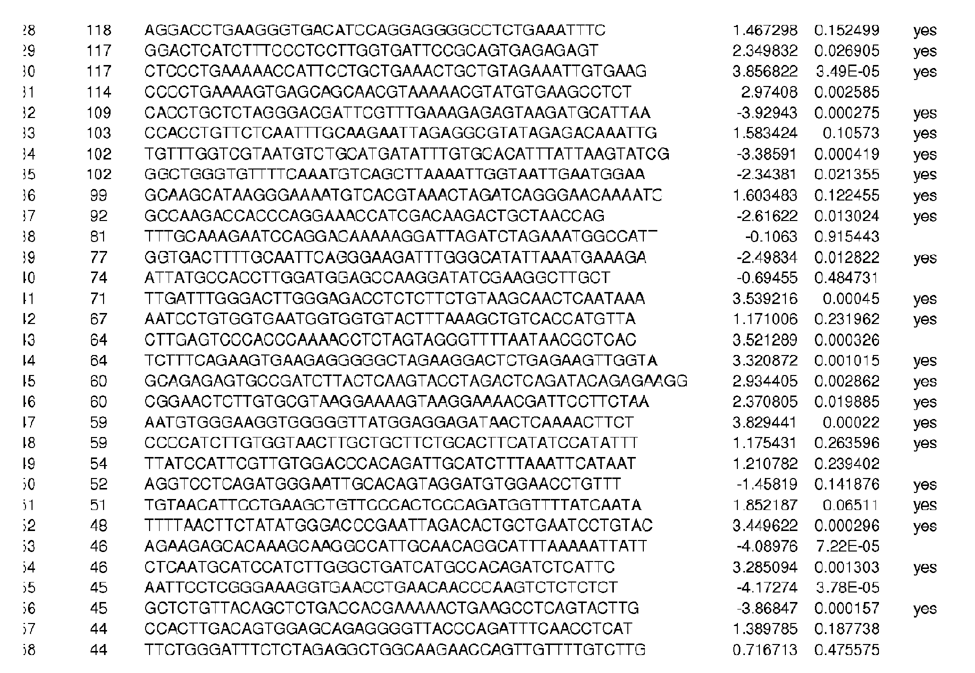

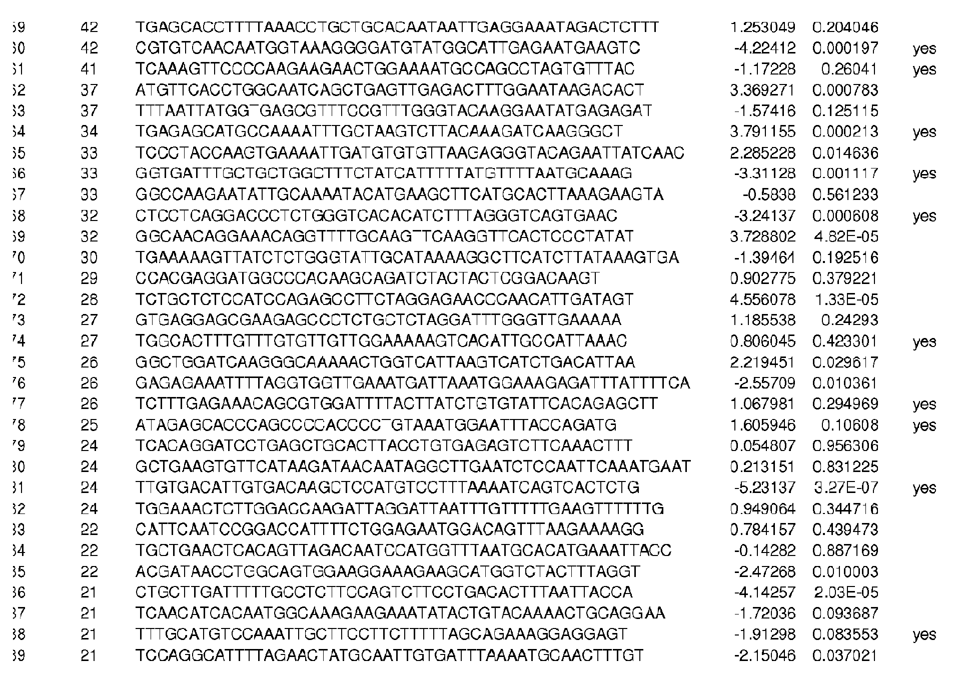

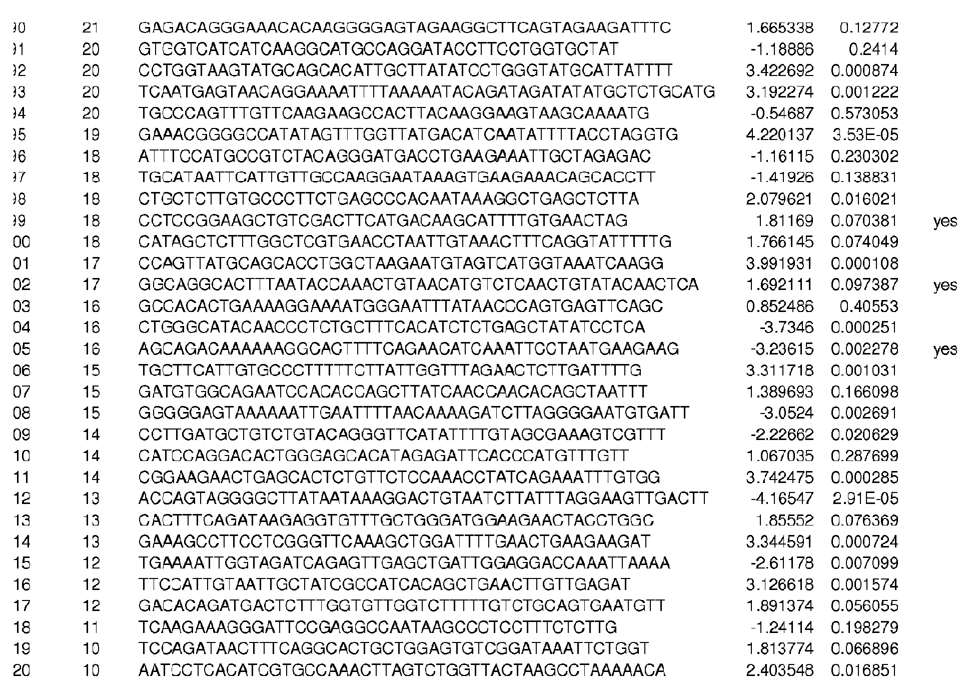

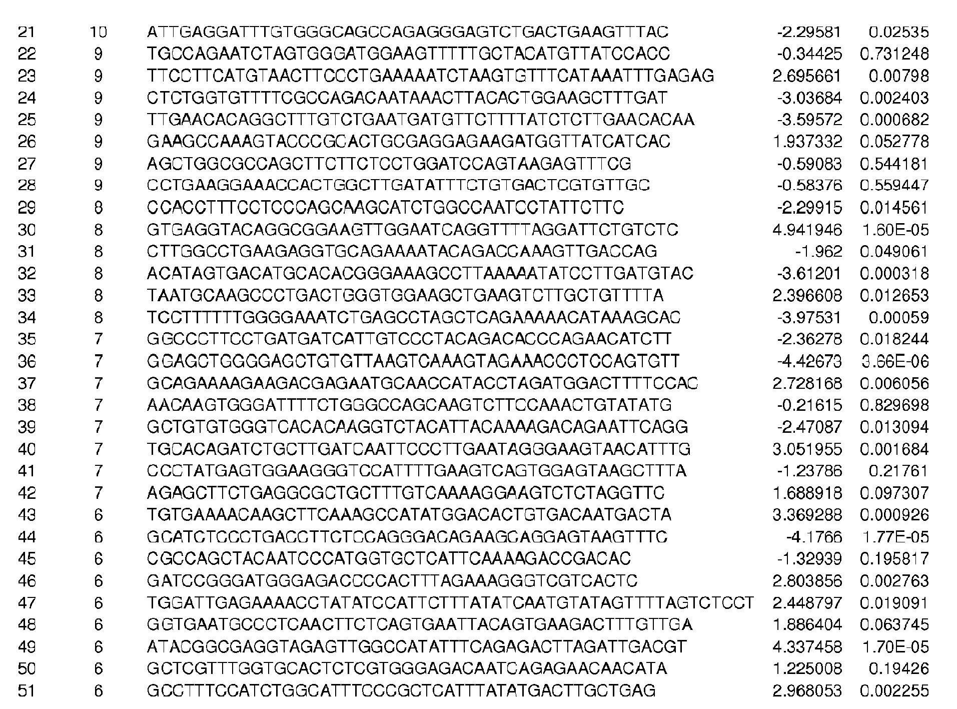

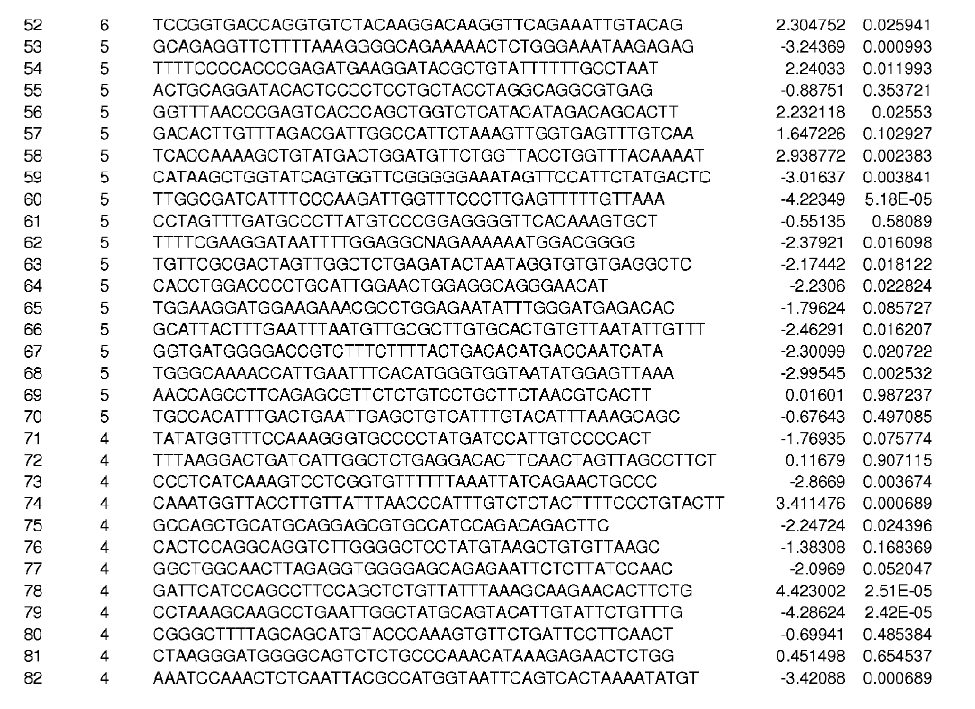

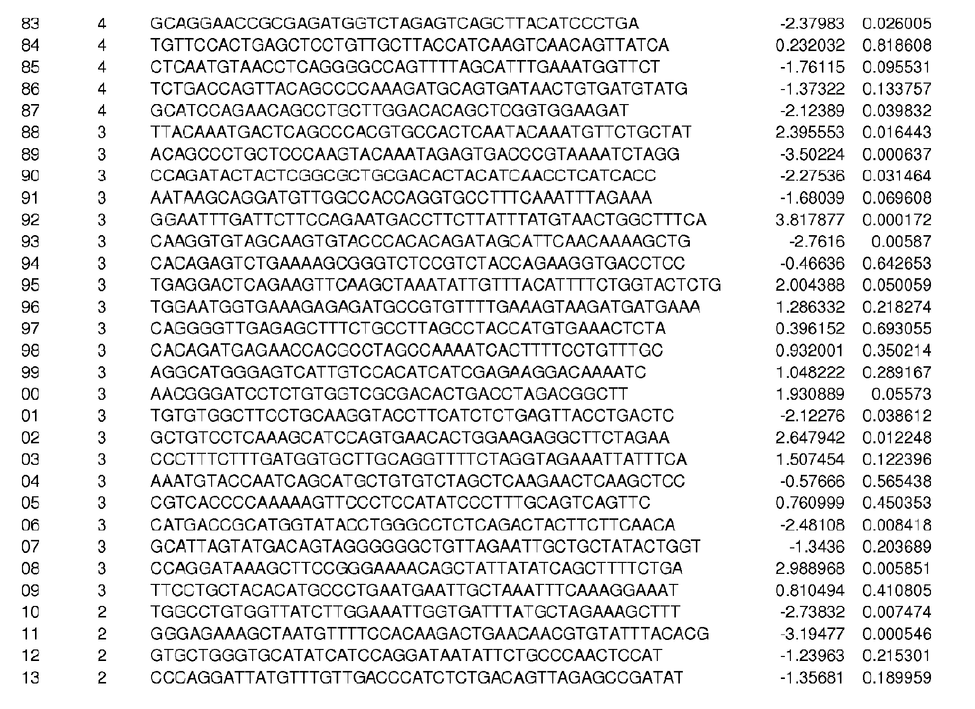

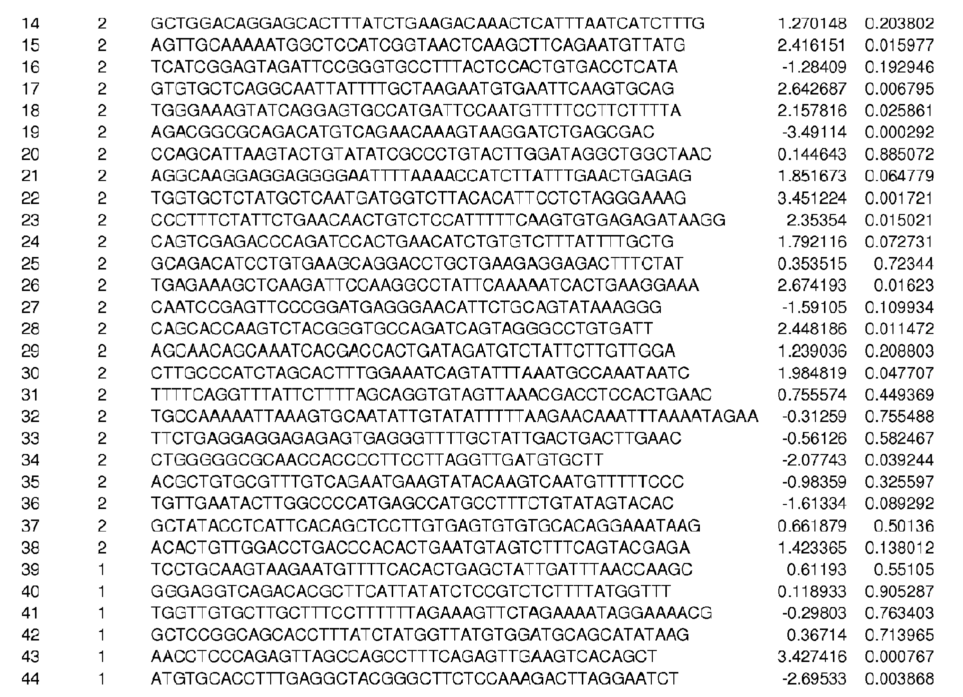

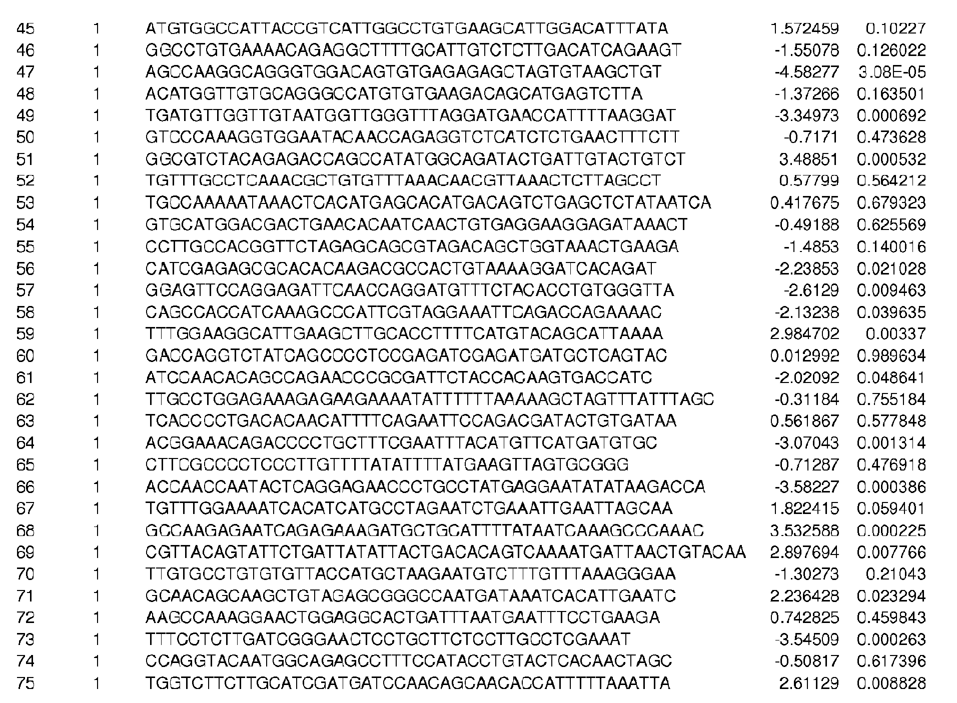

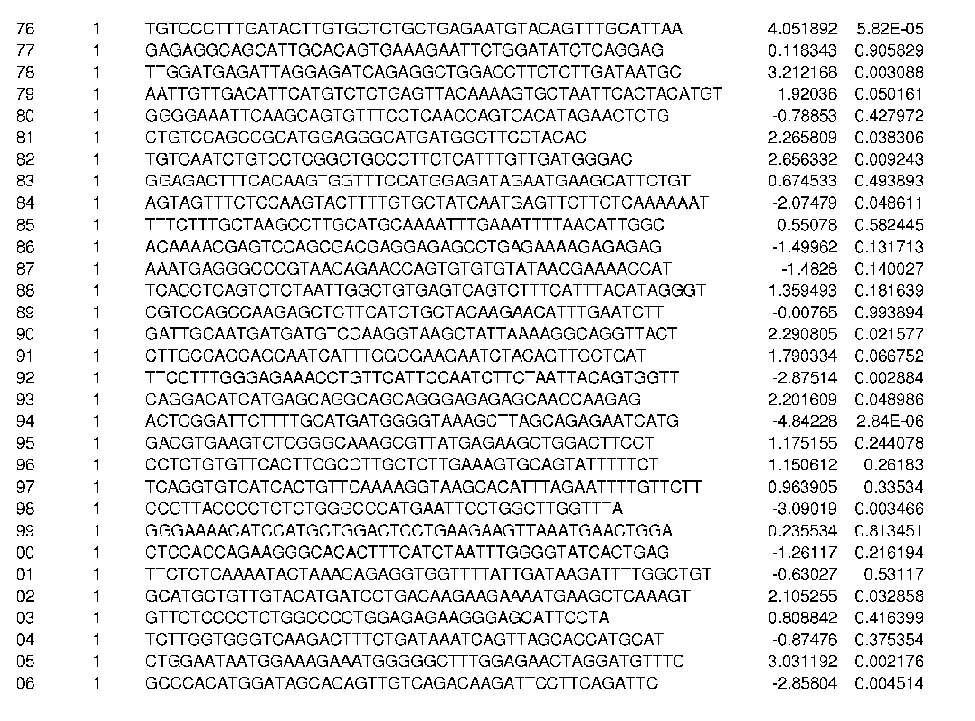

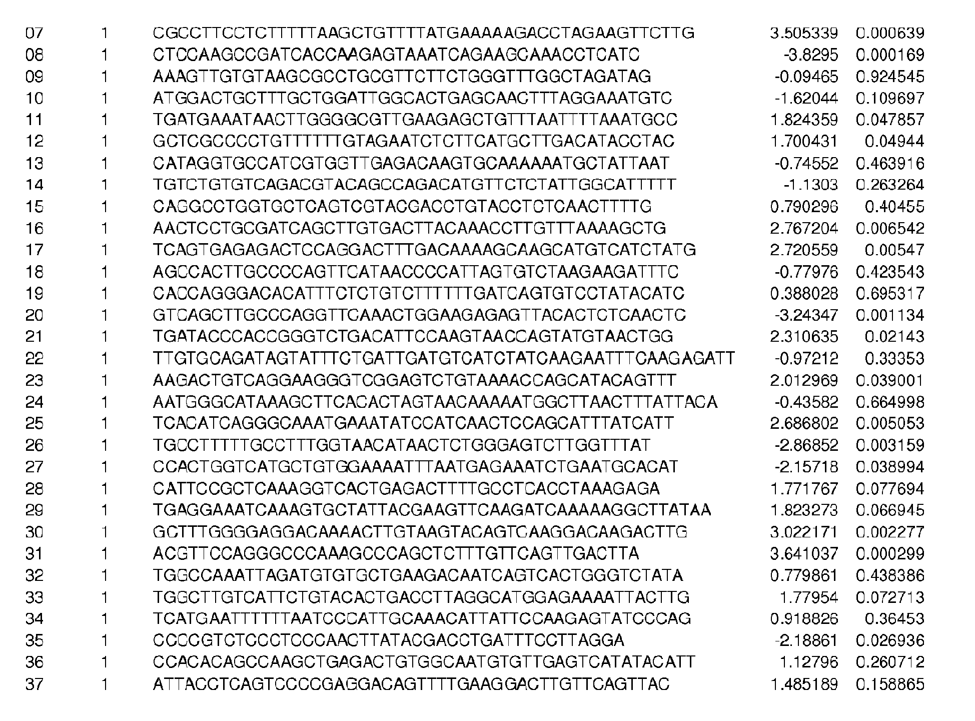

### Assay methods

To determine the (increased or decreased) expression levels of genes (expressed sequences) in the practice of the disclosure, any method known in the art may be utilized. In some embodiments, expression based on detection of RNA which hybridizes to the genes (or probe sequences) identified and disclosed herein is used. This is readily performed by any RNA detection or amplification+detection method known or recognized as equivalent in the art such as, but not limited to, reverse transcription-PCR (RT-PCR), real-time PCR, real-time RT-PCR, the methods disclosed in U.S. Patent Application 10/062,857 (filed on October 25, 2001) as well as U.S. Provisional Patent Applications 60/298,847 (filed June 15, 2001) and 60/257,801 (filed December 22, 2000), and methods to detect the presence, or absence, of RNA stabilizing or destabilizing sequences.

Alternatively, expression based on detection of DNA status may be used. Detection of the DNA of an identified gene as methylated or deleted may be used for genes that have decreased expression in correlation with a particular prostate cancer, or post-prostatectomy, outcome. This may be readily performed by PCR based methods known in the art, including, but not limited to, Q-PCR. Conversely, detection of the DNA of an identified gene as amplified may be used for genes that have increased expression in correlation with a particular prostate cancer, or post-prostatectomy, outcome. This may be readily performed by PCR based, fluorescent in situ hybridization (FISH) and chromosome in situ hybridization (CISH) methods known in the art.

Expression based on detection of a presence, increase, or decrease in protein levels or activity may also be used. Detection may be performed by any immunohistochemistry (IHC) based, blood based (especially for secreted proteins), antibody (including autoantibodies against the protein) based, exfoliate cell (from the cancer) based, mass spectroscopy based, and image (including used of labeled ligand) based method known in the art and recognized as appropriate for the detection of the protein. Antibody and image based methods are additionally useful for the localization of tumors after determination of cancer by use of cells obtained by a non-invasive procedure (such as lavage or needle aspiration), where the source of the cancerous cells is not known. A labeled antibody or ligand may be used to localize the carcinoma(s) within a patient.

One embodiment using a nucleic acid based assay to determine expression is by immobilization of one or more sequences of the genes identified herein as a polynucleotide on a solid support, including, but not limited to, a solid substrate as an array or to beads or bead based technology as known in the art. In some embodiments, the assay is DASL (cDNA-mediated Annealing, Selection, extension and Ligation) assay available from Illumina. Alternatively, solution based expression assays known in the art may also be used.

The immobilized polynucleotide probes may be unique or otherwise specific to the disclosed genes (or expressed sequences) such that the polynucleotides are capable of hybridizing to a DNA or RNA corresponding to the genes (or expressed sequences). These polynucleotides may be the full length of the genes (or expressed sequences) or be probes of shorter length (up to one nucleotide shorter than the full length sequence known in the art by deletion from the 5' or 3' end of the sequence) that are optionally minimally interrupted (such as by mismatches or inserted non-complementary basepairs) so that their hybridization with cognate DNA or RNA corresponding to the genes (or expressed seuqences) is not affected. In many embodiments, a polynucleotide probe contains sequence from the 3' end of a disclosed gene or expressed sequence. Polynucleotide probes containing mutations relative to the sequences of the disclosed genes may also be used so long as the presence of the mutations still allows hybridization to produce a detectable signal.

The immobilized polynucleotides may be used to determine the state of nucleic acid samples prepared from sample prostate cell(s) for which the outcome of the sample's subject (e.g. patient from whom the sample is obtained) is not known or for confirmation of an outcome that is already assigned to the sample's subject. Without limiting the disclosure, such a cell may be from a patient with prostate cancer, such as material removed by prostatectomy. The immobilized polynucleotide(s) need only be sufficient to specifically hybridize to the corresponding nucleic acid molecules derived from the sample under suitable conditions. While expression of even a single correlated gene may to able to provide adequate accuracy in discriminating between two prostate cancer outcomes, the disclosure includes use of expression levels from more than one gene or expressed sequence.

Therefore, the disclosure includes use of six or more, seven or more, eight or more, nine or more, ten or more, or eleven or more genes or expressed sequences disclosed herein to discriminate among outcomes. Additionally, expression levels of 12 or more, 14 or more, 16 or more, 18 or more, 20 or more, 22 or more, 24 or more, 26 or more, 28 or more, 30 or more, 32 or more, 34 or more, 36 or more, 38 or more, 40 or more, 45 or more, 50 or more, 55 or more, 60 or more, 62 or more, 65 or more, 70 or more, 75 or more, 80 or more, 85 or more, 90 or more, or 92 or more genes or expressed sequences may be assayed and used in a disclosed method. Of course additional embodiments include using 100 or more, 150 or more, 200 or more, 250 or more, 300 or more, up to the 337 in each of Table 1 and Figure 14, or the total 362 genes (or expressed sequences) as disclosed herein.

In some embodiments, the genes (expressed sequences) of the set of 62, or the set of 92, are used. In other embodiments, the genes (or expressed sequences) used are from the set of 337 in Table 1 and Figure 14, or are a combination of genes from there and those in Table 3 and Figure 15. In many cases, the combination or set of genes (or expressed sequences) used includes Gene No: 1 (FEV) or an expressed sequence which hybridizes to SEQ ID NO:1. In further embodiments, a combination includes one or more genes or expressed sequences expressed with a correlation p value of <0.0001.

Alternatively, a combination includes genes or expressed sequences with a high frequency of occurrence as disclosed herein. Non-limiting examples of such genes or expressed sequences with a frequency of more than 400, more than 350, more than 300, more than 250, more than 200, more than 150, or more than 100 as described herein. But of course combinations of one or more genes (or sequences) with a higher frequency with one or more genes (or sequences) with a lower frequency may also be used.

In embodiments where only two or a few genes are to be analyzed, the nucleic acid derived from the sample prostate cancer cell(s) may be preferentially amplified by use of appropriate primers (such as used in PCR) such that only the genes to be analyzed are amplified to reduce contaminating background signals from other expressed genes or sequences in the sample. The size of a PCR amplicon of the disclosure may be of any size, including at least or about 50, at least or about 100, at least about or 150, at least or about 200, at least or about 250, at least or about 300, at least or about 350, or at least or about 400 consecutive nucleotides, all with inclusion of the portion complementary to the PCR primers used. Of course the PCR may optionally be reverse-transcription coupled PCR (or RT-PCR in the case of RNA starting material) or quantitative PCR, such as real-time PCR, or combinations thereof. Of course RNA from the samples described herein may be prepared and used by means readily known to the skilled person.

Alternatively, and where multiple genes are to be analyzed or where very few cells (or one cell) is used, the nucleic acid from the sample may be globally amplified before hybridization to immobilized polynucleotide probes, such as on an array or microarray. Of course RNA, or the cDNA counterpart thereof may be directly labeled and used, without amplification, by methods known in the art.

The disclosure provides a more objective set of criteria, in the form of gene expression profiles of a discrete set of genes, to discriminate (or delineate) between prostate cancer, or post-prostatectomy, outcomes. In some embodiments, the assays are used to discriminate between better and poorer outcomes within 10, or about 10, years after surgical intervention to remove prostate cancer tumors. Comparisons that discriminate between outcomes after about 10, about 20, about 30, about 40, about 50, about 60, about 70, about 80, about 90, or about 100 months may also be performed.

While better and poorer cancer recurrence, metastasis and/or survival outcomes may be defined relatively in comparison to each other, a "better" outcome may be viewed as one that is better than a 50% chance of cancer recurrence and/or 50% chance of survival after about 60 months post surgical intervention to remove prostate cancer tumor(s). A "better" outcome may also be a better than about 60%, about 70%, about 80% or about 90% cancer recurrence and/or chance of survival about 60 months post surgical intervention. A "poorer" outcome may be viewed as a 50% or more chance of cancer recurrence and/or less than 50% chance of survival after about 60 months post surgical intervention to remove prostate cancer tumor(s).

The disclosed methods may also be used with solid tissue material. For example, a solid biopsy may be collected and prepared for visualization followed by determination of expression of two or more genes or expressed sequences identified herein to determine the prostate cancer, or post-prostatectomy, outcome. One means is by use of *in situ* hybridization with polynucleotide or protein identifying probe(s) for assaying expression of said gene(s).

In some embodiments, the detection of gene expression from the samples may be by use of a single microarray able to assay gene expression from some or all genes disclosed herein for convenience and accuracy.

### Additional embodiments

Other uses of the disclosure include providing the ability to identify prostate cancer cell samples as correlated with particular prostate cancer survival or recurrence outcomes for further research or study. This provides a particular advantage in many contexts requiring the identification of cells based on objective genetic or molecular criteria.

The materials for use in the methods of the present disclosure are ideally suited for preparation of kits produced in accordance with well known procedures. The disclosure thus provides kits comprising agents for the detection of expression of the disclosed genes and sequences for identifying prostate cancer, or post-prostatectomy, outcomes. Such kits optionally comprise the agent with an identifying description or label or instructions relating to their use in the methods of the present disclosure, is provided. Such a kit may comprise containers, each with one or more of the various reagents (typically in concentrated form) utilized in the methods, including, for example, pre-fabricated microarrays, buffers, the appropriate nucleotide triphosphates (e.g., dATP, dCTP, dGTP and dTTP; or rATP, rCTP, rGTP and UTP), reverse transcriptase, DNA polymerase, RNA polymerase, and one or more primer complexes of the present disclosure (e.g., appropriate length poly(T) or random primers linked to a promoter reactive with the RNA polymerase). A set of instructions will also typically be included.

The methods provided by the disclosure may also be automated in whole or in part. All aspects of the disclosure may also be practiced such that they consist essentially of a subset of the disclosed genes to the exclusion of material irrelevant to the identification of prostate cancer recurrence, metastasis, and/or survival outcomes via a cell containing sample.

Having now generally provided the disclosure, the same will be more readily understood through reference to the following examples which are provided by way of illustration, and are not intended to be limiting of the disclosure, unless specified.

### EXAMPLES

### Example I: Clinical specimen collection and clinicopathological parameters.

Prostate samples with PSA and other patient outcome data from radical prostatectomies in 1993-1995 were used to discover sets of genes (expressed sequences), the expression levels of which correlate with clinical prostate cancer, or post-prostatectomy, outcome. Other samples were used to test, or verify the predictive or prognostic ability of the identified gene sets.

The characteristics of the patient profiles corresponding to the samples are shown in Table 2.

**Table 2**

| **Factor Description** | | **Train (n=124)** | **Test (n=67)** | **All (n=191)** |
|---|---|---|---|---|
| **Age** | Mean | 61.8 | 62.7 | 62.1 |
| | Range | 45-77 | 50-78 | 45-78 |
| **Gleason Score** | <=6 | 42 (34%) | 27 (40%) | 69 (36%) |
| | 7 | 65 (52%) | 32 (48%) | 97 (51%) |
| | >=8 | 17 (14%) | 8 (12%) | 25 (13%) |
| **AJCC Stage** | II | 95 (77%) | 49 (73%) | 144 (75%) |
| | III | 29 (23%) | 18 (27%) | 47 (25%) |
| **Surgical Margin** | positive | 51 (41%) | 26 (39%) | 77 (40%) |
| | negative | 73 (59%) | 41 (61%) | 114 (60%) |
| **BCF Follow-up (years)** | Mean | 6.9 | 7.7 | 7.2 |
| | Range | 0.2-11.5 | 1.6-11.4 | 0.2-11.5 |
| **BCF Event** | No | 71 (57%) | 43 (64%) | 114 (60%) |
| | Yes | 53 (43%) | 24 (36%) | 77 (40%) |
| **MFS Follow-up (years)** | Mean | 9 | 8.7 | 8.9 |
| | Range | 0.2-13.3 | 0.3-12.7 | 0.2-13.3 |
| | Unknown | 1 | 1 | 2 |
| **Metastasis Event** | No | 112 (91%) | 62 (94%) | 174 (92%) |
| | Yes | 11 (9%) | 4 (6%) | 15 (8%) |
| | Unknown | 1 | 1 | 2 |
| **Pre-op PSA** | Mean | 8.4 | 8.9 | 8.6 |
| | Range | 1.1-37.2 | 1.7-31.8 | 1.1-37.2 |
| | Unknown | 49 | 20 | 69 |

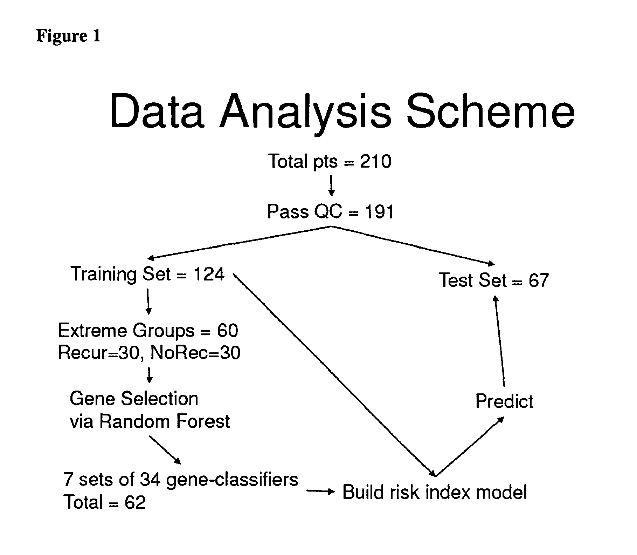

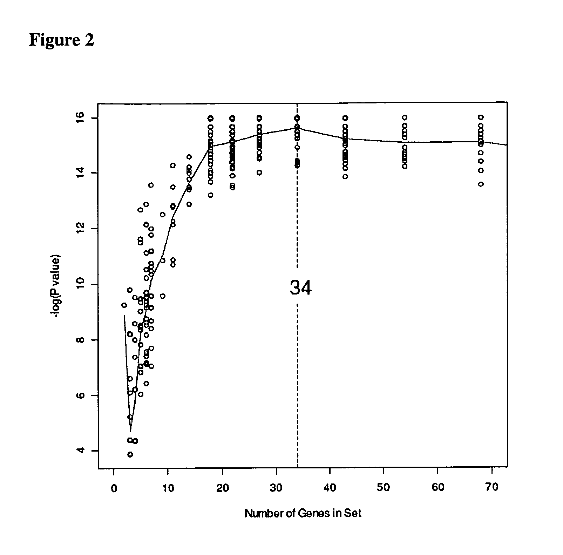

The methodology for using the patient samples for this identification is schematically illustrated in Figure 1. Briefly, 191 patient samples were selected from an initial set of 210. The 191 were divided into a training set (124 samples) and a test set (67 samples), where the former was used to identify gene sets via the Random Forests^{™} algorithm. Representative results with 500 independent runs (gene sets) obtained by use of the algorithm are shown in Figure 2 as a plot of the number of genes (expressed sequences) in a set versus the log of unadjusted P values (equivalent to log rank test). Figure 2 shows the performance of these 500 sets, containing various numbers of genes (expressed sequences) and their ability to accurately classify samples (and so patients) as high or low risk of cancer recurrence and/or metastasis with significance as indicated by the observed unadjusted P values. The genes (expressed sequences) in the 500 sets reflect 337 unique (non-duplicate) nucleic acid molecules that are summarized in Table 1 above and Figure 14 herein.

Seven sets of 34 genes generated by the algorithm were reviewed and found to reflect 62 non-duplicate molecules which are identified in Table 1 above (see "set of 62" column therein, which may be cross referenced with Figure 14 via Gene No. (in comparison to SEQ ID NO) and frequency of occurrence among the 337 entries. The expression levels of these 62 genes (expressed sequences) are discussed herein as a non-limiting, and representative, example of gene sets, including the additional sets shown in Figure 2 and as disclosed herein. So expression levels of the 62 genes in prostate cancer cell samples were used as a risk index (predictor) of outcome in the test set as illustrated in Figure 1.

### Example II: Identification of subtypes with different patient outcomes

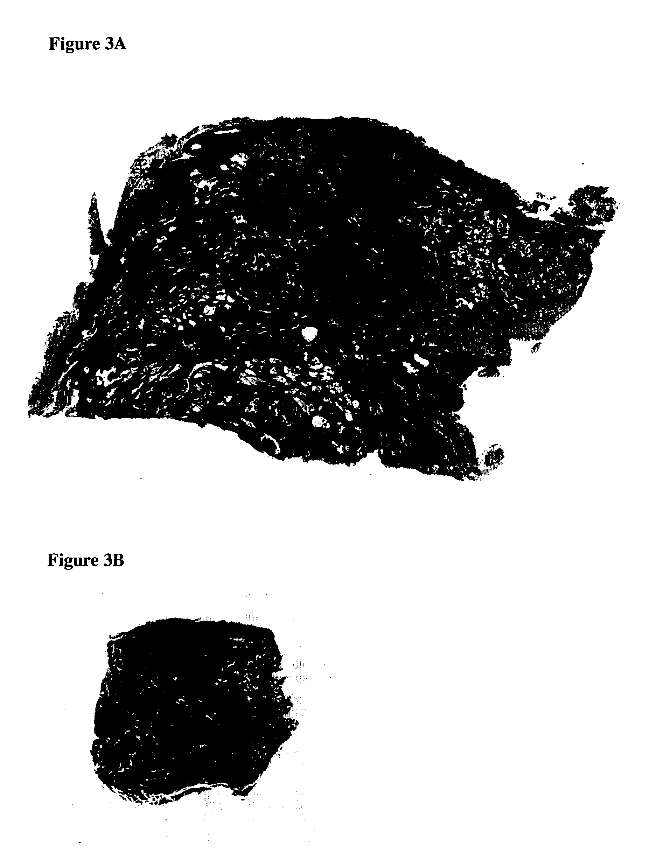

Gene selection was used with a training set of 124 samples to identify genes that predicted outcomes. See Figure 1 and Table 2. The identified genes were then used to predict outcomes in a test set of 67 samples processed by isolation of cancerous cells (see Figures 3A and 3B). The predicted outcomes were compared to the documented outcomes for the patients from whom the samples were obtained.

As a representative example, gene-specific oligonucleotide probes are immobilized, such as on a microarray and then hybridized to Cy5-labeled sample RNA and Cy3-labeled reference RNA in a co-hybridization reaction at 65°C in 1x hybridization buffer. The immobilized label is washed at 37°C with 0.1x SSC/0.005% Triton X-102. Image analysis is performed using image analysis software. Raw Cy5/Cy3 ratios is normalized using intensity-dependent non-linear regression.

Normalized Cy5/Cy3 ratios from all samples are median (or mean) centered for each gene. Identification and permutation testing for significance of differential gene expression is performed using appropriate software, such as Cox regression analysis and Random Forests™. Disease free survival is calculated from the date of diagnosis. Events are scored as the first recurrence or distant metastasis. Survival curves were calculated by the Kaplan-Meier estimates and compared by *log-*rank tests.

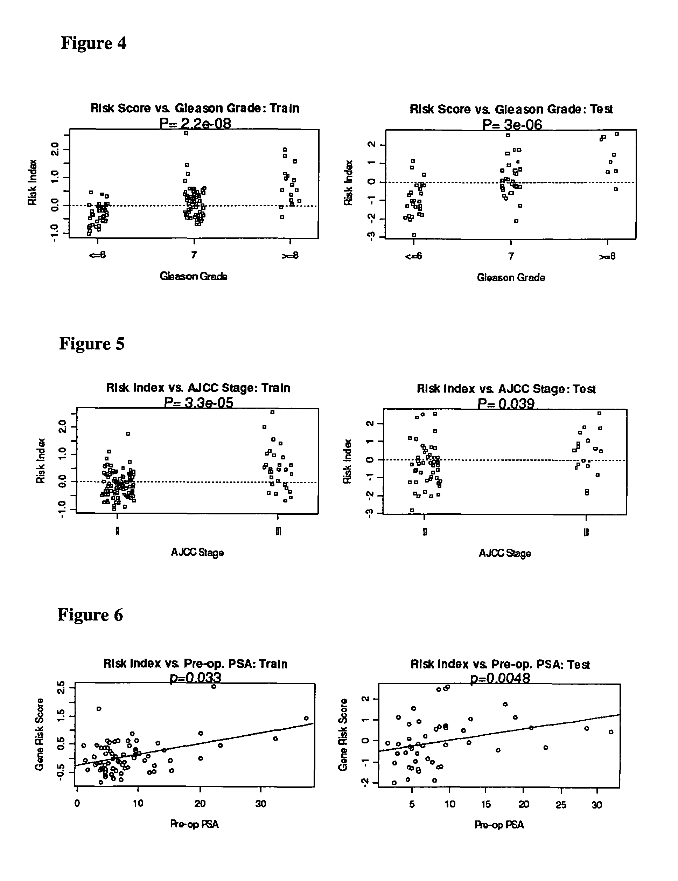

Figures 4-6 show the performance of a representative Risk Index (or Risk Score) using the disclosed 62 gene set as a combination in comparison to known factors used for assessing prostate cancer samples and patients. The factors are Gleason Grade (or Gleason Score), AJCC staging system for prostate cancer, and pre-operation (pre-surgical intervention) PSA (prostate serum antigen) levels. These comparisons show that a gene expression profile of the disclosure correlates with these other factors and so may be used in combination with any one or more of these factors or used alone to recapitulate information provided by each of these factors.

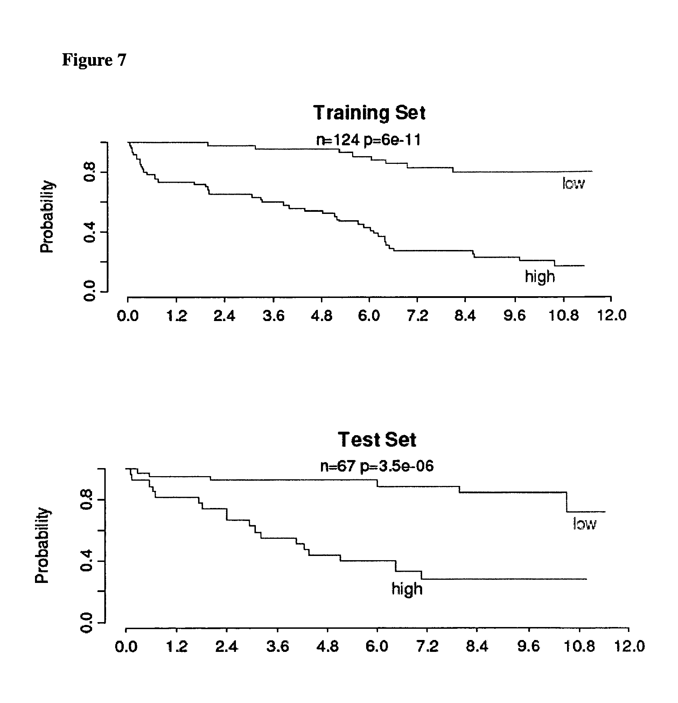

Expression levels of the 62 genes (expressed sequences) were also found to be able to segregate the 124 samples of the training set and the 67 samples of the test set into "high" and "low" risk classifications (see Figure 7).

### Example III: Identification of subtypes within different prostate cancer grades

In combination with Figure 7, Figure 4 demonstrates that a Risk Score (or Risk Index) in the "high" and "low" subclasses, based on expression levels of the representative 62 gene set, may be used to classify samples (and so patients) in a manner consistent with Gleason Grade, where the higher the grade, the greater the likelihood of a Risk Score above a value of zero, and so a higher risk of a poor outcome. The zero value represents the mean and/or the median value for the Score across all prostate cancer samples in the set. The "high risk" classification is based on a Risk Score above zero, and the "low risk" classification is based on values below zero. As a result, the Risk Score (or Risk Index) may be used to classify samples, and so patients, with a Gleason Grade of ≤6 or 7 (or even ≥8) into "high" and "low" risk groups as described herein. See Figure 8.

This demonstrates that the Risk Score (or Risk Index) may be used to classify samples, and so patients, independent from use of the Gleason Grade. Alternatively, the two analyses may be used together, optionally in series, such as determination of the Gleason Grade followed by determination of the Risk Score in samples (and so patients) with a Grade of ≤6 or 7 (or even ≥8). The combined use is particularly advantageous to "rule in" subjects at high risk for appropriate or further clinical treatment, such as by chemotherapy or radiation or cryotherapy as known to the skilled clinician and to "rule out" subjects at low risk so that they are not unnecessarily subject to treatments with undesirable side effects.

### Example IV: Identification of subtypes within different prostate cancer stages

In combination with Figure 7, Figure 5 demonstrates that a Risk Score (or Risk Index) in the "high" and "low" subclasses, based on expression levels of the representative 62 gene set, may be used to classify samples (and so patients) in a manner consistent with AJCC prostate cancer stage, where stage III is more likely to correlate with a Risk Score above a value of zero, and so higher risk of a poor outcome outcome is present. The zero value again represents the mean and/or the median value for the Score across all prostate cancer samples in the set. The "high risk" group is again based on a Risk Score above zero, and the "low risk" group is based on values below zero. As a result, the Risk Score (or Risk Index) may be used to classify samples, and so patients, with an AJCC stage II or III classification into "high" and "low" risk groups as described herein.

This demonstrates that the Risk Score (or Risk Index) may be used to classify samples, and so patients, independent from use of the AJCC stage assessment. Alternatively, the two analyses may be used together, optionally in series, such as determination of the AJCC stage followed by determination of the Risk Score in samples (and so patients) with stage II or III prostate cancer. The combined use is particularly advantageous to "rule in" subjects at high risk for appropriate clinical treatment, as known to the skilled clinician, and to "rule out" subjects at low risk so that they may reasonably avoid treatments with undesirable side effects. Combined used may also alter the classification of a patient or subject as suited for "watchful waiting."

### Example V: Combination with pre-operative PSA analysis

While the Gleason Grade and AJCC stage analysis both require use of prostate cancer cell samples, the consistencies in Figure 6 between Risk Score (or Risk Index) in the "high" and "low" subclasses, based on expression levels of the representative 62 gene set, indicates that a pre-operative PSA value should be considered in combination with a Risk Score as described herein. The combination may be in series, such as determination of the pre-operative PSA value followed by determination of the Risk Score in samples (and so patients) with PSA values of 30 or more, 25 or more, 20 or more, 15 or more, 10 or more, or 5 or more.

### Example VI: Identification of genes by Cox regression analysis

Cox regression analysis was used to identify 92 genes (or expressed sequences) the expression levels of which are capable of classifying prostate cancer cell containing samples as described above. Sixty-seven (67) of these 92 genes (or expressed sequences) are common to the 337 genes (or expressed sequences) in Figure 14 and Table 1 (see "Cox92" column in Figure 14 or Table 1, which can be cross referenced to each other by Gene No. (versus SEQ ID NO) and frequency. The remaining twenty-five (25) genes (or expressed sequences) out of the 92 are listed in Figure 15 and Table 3 below, which may be cross referenced to each other by Gene No. (in comparison to SEQ ID NO) as well as z and p values.

**Table 3**

| SEQ ID NO: | Probe_Sequence | Z | p |
|---|---|---|---|
| 338 | GATGCACCAGGCGAGGAAACGAGACCTCTTTCGTTCCTTCTAG | -4.999253 | 4.18E-06 |
| 339 | ATGCCTGAGGCATGAGTGACTGTGCATTTGAGATAGTTTTCCCTAT | -4.834638 | 4.97E-06 |
| 340 | TGAAAAAGAACAAGAGCTGGACACATTAAAAAGAAAGAGTCCATCAGATT | 4.3810131 | 2.31 E-05 |
| 341 | CAGAAGGACCTGGGGGATGGCGTGTATGGCTTCGAGTATTACC | -4.181005 | 2.89E-05 |
| 342 | AGGGCTGTCATCAACATGGATATGACATTTCACAACAGTGACTAG | -3.804427 | 5.90E-05 |
| 343 | TGGCTTTGCACAGAACCAGCTAGTTATTTGGAAGTACCCAACC | 4.2565288 | 5.95E-05 |
| 344 | TGTGGGTTAGCATCAAGTTCTCCCCAGGGTAGAATTCAATCAGAGCT | -4.211286 | 8.06E-05 |
| 345 | CAATGCAAAAAGTATTCGCTGCTGTTTACATTAGAAATCACTTCCAGC | -3.829636 | 8.59E-05 |
| 346 | TGTACAGTGGAACCATGTGACCATGTCTTGTGCTTGCAATATAGAAA | 3.7423758 | 0.0001308 |
| 347 | GGCAAATCCTTCAAGCAGGGATAAAAGTCGATCTTCAAACATTAACTT | -3.604833 | 0.0001884 |
| 348 | GGGTTTGGGGGAGATTTTACTCCTTTCTTCAACAACTATTCACTGGA | -3.554167 | 0.0003955 |
| 349 | TTTTAGGCCTTTGGGGGAATTGATTTTTATCCACAGGTAGAAAATG | -3.576323 | 0.0004075 |
| 350 | CTATGCAGGAAAATAGCACCCCCCGTGAGGACTAATCCAGATACATC | -3.553166 | 0.0004276 |
| 351 | AGGTATGGCCTCACAAGTCTTGGTCTACCCACCATATGTTTATCAAA | -3.464213 | 0.0006149 |
| 352 | AGGAGGTGTCAGACTGCTGAAGCCGACTCTGAAAGTGATCATGAAGT | 3.5820846 | 0.0006381 |
| 353 | GAGGGCATGTTGTCCATATCCCTGTGGAATACAGACCGTGTAACT | 3.4420595 | 0.0006641 |
| 354 | TCTAATGTGCACGGTGTGACTGGCAGAGTGAGTTTAAAAGCTTTACGA | -3.230789 | 0.0007048 |
| 355 | TGTTGCCAAGCTAGTCTACAAAGCATCTGATTTTGGAAGTACATGGAAT | 3.6130547 | 0.0007484 |
| 356 | TTACACCTTTCCCCCCTGAAATGTATAGAATCCATTTGTCATCAGG | -3.434091 | 0.0007518 |
| 357 | AATTGGTGAACAAAAAATGCCCAAGGCTTCTCATGTCTTTATTCTG | -3.341571 | 0.0007655 |
| 358 | GGAGAATTCTTTAGGTTGTCCCCTAAAGATTCTGAAAAAGAGAATCAGA | 3.3060176 | 0.0007756 |
| 359 | GTAGCCTCACCATTAGTGGCAGCATCATGTAACTGAGTGGACTGTG | 3.8470148 | 0.0007772 |
| 360 | TTTGCTCACAAGCCATATTGGCCCGATTAGTGGTACTGTCTGACTC | 3.2305915 | 0.000808 |
| 361 | TGAGCTTACAACAGGTCTCGAGCTGGTGGACTCCTGTATTAGGTCACT | 3.2987578 | 0.000825 |
| 362 | GCATGCAGATGTCAAGGCAGTTAGGAAGTAAATGGTGTCTTGTAGA | 3.2511294 | 0.0008645 |

## Claims

1. A method to determine the risk of prostate cancer recurrence in a subject, said method comprising
assaying a sample comprising prostate cancer cells from said subject for the expression levels of six or more genes selected from Gene Nos: 1 to 362 in Figures 14 and 15,
determining, as an aggregated value, a sum of the expression levels of said six or more genes, and
determining the risk of prostate cancer recurrence in said subject based upon said aggregated value,
wherein the expression levels are correlated with
a low risk of cancer recurrence, or
a high risk of cancer recurrence.

2. The method of claim 1 wherein said method further comprises selecting a treatment for a subject with the determined cancer recurrence.

3. The method of claim 1 or 2 wherein said assaying comprises preparing RNA from said sample.

4. The method of claim 3 wherein said RNA is used for PCR.

5. The method of claim 3 wherein said assaying comprises using an array.

6. The method of claim 1 or 2 wherein said sample is dissected from tissue removed during prostatectomy.

7. The method of claim 4 wherein said PCR is RT-PCR, optionally real time RT-PCR.

8. The method of claim 1 or 2 or 3 or 4 or 5 or 6 or 7 wherein said expression levels are correlated with a p value of <0.0001.

9. The method of claim 1 or 2 or 3 or 4 or 5 or 6 or 7 wherein said sample comprises isolated prostate cancer cells.

10. The method of claim 2 wherein said treatment comprises chemotherapy or radiation therapy.

11. The method of claim 1 further comprising
i) determining the grade of prostate cancer in said sample,
ii) determining the stage of prostate cancer in said sample, or
iii) both;
wherein i), ii) or both i) and ii) are optionally performed before determining the risk of prostate cancer recurrence in said subject.

12. The method of claim 11 wherein determining prostate cancer grade comprises determination of a Gleason Score or the AJCC stage or
wherein the method comprises determining prostate cancer grade by a Gleason Score and determining prostate cancer stage according to AJCC stage to produce a multivariate analysis for determining the risk of prostate cancer recurrence in said subject.

13. The method of claim 1 further comprising determining prostate serum antigen (PSA) levels in said subject, optionally before a prostatectomy which is used to provide said sample comprising prostate cancer cells.

14. The method of claim 1 wherein expression levels of 8 or more, 10 or more, 12 or more, 14 or more, 16 or more, 18 or more, 20 or more, 22 or more, 24 or more, 26 or more, 28 or more, 30 or more, 32 or more, 34 or more, 36 or more, 38 or more, 40 or more, 45 or more, 50 or more, 55 or more, 60 or more, 65 or more, 70 or more, or 92 or more genes are assayed.

15. The method of claim 1 wherein said assaying comprises determining the expression level of Gene No. 1, optionally via use of SEQ ID NO: 1 as a probe.

16. A method to determine the risk of developing metastasis of prostate cancer in a subject, said method comprising
assaying a sample comprising prostate cancer cells from said subject for the expression levels of six or more genes selected from Gene Nos: 1 to 362 in Figures 14 and 15;
determining, as an aggregated value, a sum of the expression levels of said six or more genes, and
determining the risk of developing metastasis of prostate cancer in said subject based upon said aggregated value,
wherein the expression levels are correlated with low risk of developing metastasis of prostate cancer, or
a high risk of developing metastasis of prostate cancer.

17. The method of any preceding claim, wherein the sample comprising prostate cancer cells is a needle biopsy or other biopsy.

## Patentansprüche

1. Verfahren zur Bestimmung des Risikos für Prostatakrebs-Rezidiv bei einem Subjekt, wobei das Verfahren umfasst:
Analysieren einer Probe, die Prostatakrebszellen umfasst, von dem Subjekt auf die Expressionslevel von sechs oder mehr Genen, ausgewählt aus Gen Nrn. 1 bis 362 in Figuren 14 und 15,
Bestimmen, als aggregierten Wert, eine Summe der Expressionslevel der sechs oder mehr Gene und
Bestimmen des Risikos für Prostatakrebs-Rezidiv bei dem Subjekt auf der Basis des aggregierten Wertes,
wobei die Expressionslevel mit
einem geringen Risiko für Krebsrezidiv oder
einem hohen Risiko für Krebsrezidiv korreliert werden.

2. Verfahren gemäß Anspruch 1, wobei das Verfahren außerdem Auswählen einer Behandlung für ein Subjekt mit dem bestimmten Krebsrezidiv umfasst.

3. Verfahren gemäß Anspruch 1 oder 2, wobei das Analysieren Präparieren von RNA aus der Probe umfasst.

4. Verfahren gemäß Anspruch 3, wobei die RNA für PCR verwendet wird.

5. Verfahren gemäß Anspruch 3, wobei das Analysieren die Verwendung eines Arrays umfasst.

6. Verfahren gemäß Anspruch 1 oder 2, wobei die Probe aus Gewebe, das während Prostatektomie entfernt worden ist, disseziert wird.

7. Verfahren gemäß Anspruch 4, wobei die PCR RT-PCR, gegebenenfalls Echtzeit-RT-PCR ist.

8. Verfahren gemäß Anspruch 1 oder 2 oder 3 oder 4 oder 5 oder 6 oder 7, wobei die Expressionslevel mit einem p-Wert von < 0,0001 korreliert sind.

9. Verfahren gemäß Anspruch 1 oder 2 oder 3 oder 4 oder 5 oder 6 oder 7, wobei die Probe isolierte Prostatakrebszellen umfasst.

10. Verfahren gemäß Anspruch 2, wobei die Behandlung Chemotherapie oder Strahlentherapie umfasst.

11. Verfahren gemäß Anspruch 1, außerdem umfassend:
i) Bestimmen des Prostatakrebs-Grades in der Probe,
ii) Bestimmen des Prostatakrebs-Stadiums in der Probe oder
iii) beides;
wobei i), ii) oder beides, i) und ii), gegebenenfalls vor Bestimmen des Risikos von Prostatakrebs-Rezidiv bei dem Subjekt durchgeführt werden.

12. Verfahren gemäß Anspruch 11, wobei ein Bestimmen des Prostatakrebs-Grades Bestimmen des Gleason-Scores oder des AJCC-Stadiums umfasst oder
wobei das Verfahren Bestimmen des Prostatakrebs-Grades durch den Gleason-Score und Bestimmen des Prostatakrebs-Stadiums gemäß AJCC-Stadium umfasst, um eine Multivariantenanalyse zur Bestimmung des Risikos für Prostatakrebs-Rezidiv bei dem Subjekt zu erstellen.

13. Verfahren gemäß Anspruch 1, außerdem umfassend Bestimmen der Prostataserumantigen (PSA)-Level bei dem Subjekt, gegebenenfalls vor einer Prostatektomie, die eingesetzt wird, um die Probe bereitzustellen, die Prostatakrebszellen umfasst.

14. Verfahren gemäß Anspruch 1, wobei Expressionslevel von 8 oder mehr, 10 oder mehr, 12 oder mehr, 14 oder mehr, 16 oder mehr, 18 oder mehr, 20 oder mehr, 22 oder mehr, 24 oder mehr, 26 oder mehr, 28 oder mehr, 30 oder mehr, 32 oder mehr, 34 oder mehr, 36 oder mehr, 38 oder mehr, 40 oder mehr, 45 oder mehr, 50 oder mehr, 55 oder mehr, 60 oder mehr, 65 oder mehr, 70 oder mehr oder 92 oder mehr Genen analysiert werden.

15. Verfahren gemäß Anspruch 1, wobei das Analysieren Bestimmen des Expressionslevels von Gen Nr. 1, gegebenenfalls durch Verwendung von SEQ ID NO:1 als Sonde, umfasst.

16. Verfahren zur Bestimmung des Risikos für die Entwicklung von Metastasen von Prostatakrebs bei einem Subjekt, wobei das Verfahren umfasst:
Analysieren einer Probe, die Prostatakrebszellen umfasst, von dem Subjekt auf die Expressionslevel von sechs oder mehr Genen, ausgewählt aus Genen Nrn. 1 bis 362 in Figuren 14 und 15;
Bestimmen, als aggregierten Wert, die Summe der Expressionslevel der sechs oder mehr Gene und
Bestimmen des Risikos für die Entwicklung von Metastaten von Prostatakrebs in dem Subjekt auf der Basis des aggregierten Wertes,
wobei die Expressionslevel mit geringem Risiko für die Entwicklung von Metastasen von Prostatakrebs oder
einem hohen Risiko für die Entwicklung von Metastasen von Prostatakrebs korreliert werden.

17. Verfahren gemäß einem vorangehenden Anspruch, wobei die Probe, die Prostatakrebszellen umfasst, eine Nadelbiopsie oder eine andere Biopsie ist.

## Revendications

1. Procédé visant à déterminer le risque de récidive d'un cancer de la prostate chez un sujet, lequel procédé comporte :
- le fait de mesurer, sur un échantillon comprenant des cellules cancéreuses de prostate issues dudit sujet, les niveaux d'expression de six gènes ou plus, choisis parmi les Gènes N° 1 à 362 indiqués dans les Figures 14 et 15,
- le fait de déterminer, en tant qu'indice global, la somme des niveaux d'expression desdits six gènes ou plus,
- et le fait de déterminer le risque de récidive d'un cancer de la prostate chez ledit sujet, en se basant sur cet indice global,
étant entendu que les niveaux d'expression sont corrélés
soit à un faible risque de récidive de cancer,
soit à un haut risque de récidive de cancer.

2. Procédé conforme à la revendication 1, lequel procédé comprend en outre le fait de choisir un traitement pour un sujet présentant le risque de récidive de cancer déterminé.

3. Procédé conforme à la revendication 1 ou 2, dans lequel ladite mesure comprend le fait de préparer un ARN à partir dudit échantillon.

4. Procédé conforme à la revendication 3, dans lequel ledit ARN est utilisé pour une PCR.

5. Procédé conforme à la revendication 3, dans lequel ladite mesure comprend le fait d'utiliser un réseau.

6. Procédé conforme à la revendication 1 ou 2, pour lequel ledit échantillon a été obtenu par dissection à partir de tissu prélevé au cours d'une prostatectomie.

7. Procédé conforme à la revendication 4, dans lequel ladite PCR est une RT-PCR, et en option, une RT-PCR en temps réel.

8. Procédé conforme à la revendication 1 ou 2 ou 3 ou 4 ou 5 ou 6 ou 7, dans lequel lesdits niveaux d'expression sont corrélés avec une valeur de p inférieure à 0,0001.

9. Procédé conforme à la revendication 1 ou 2 ou 3 ou 4 ou 5 ou 6 ou 7, dans lequel ledit échantillon comprend des cellules cancéreuses de prostate isolées.

10. Procédé conforme à la revendication 2, dans lequel ledit traitement comprend une chimiothérapie ou une radiothérapie.

11. Procédé conforme à la revendication 1, qui comporte en outre
i) le fait de déterminer le grade du cancer de la prostate, dans ledit échantillon,
ii) ou le fait de déterminer le stade du cancer de la prostate, dans ledit échantillon,
iii) ou ces deux déterminations,
étant entendu que l'étape (i), l'étape (ii) ou les deux étapes (i) et (ii) est ou sont, en option, effectuée(s) avant la détermination du risque de récidive du cancer de la prostate chez ledit sujet.

12. Procédé conforme à la revendication 11, dans lequel le fait de déterminer le grade du cancer de la prostate comprend la détermination d'une cote de Gleason ou du stade AJCC,
ou lequel procédé comporte le fait de déterminer le grade du cancer de la prostate à l'aide d'une cote de Gleason et le fait de déterminer le stade du cancer de la prostate d'après le stade AJCC, afin de faire une analyse multivariable pour déterminer le risque de récidive du cancer de la prostate chez ledit sujet.

13. Procédé conforme à la revendication 1, qui comporte en outre le fait de déterminer des taux d'APS (antigène prostatique sérique) chez ledit sujet, en option avant une prostastectomie qui est utilisée pour fournir ledit échantillon comprenant des cellules cancéreuses de prostate.

14. Procédé conforme à la revendication 1, dans lequel on mesure les niveaux d'expression de 8 gènes ou plus, de 10 gènes ou plus, de 12 gènes ou plus, de 14 gènes ou plus, de 16 gènes ou plus, de 18 gènes ou plus, de 20 gènes ou plus, de 22 gènes ou plus, de 24 gènes ou plus, de 26 gènes ou plus, de 28 gènes ou plus, de 30 gènes ou plus, de 32 gènes ou plus, de 34 gènes ou plus, de 36 gènes ou plus, de 38 gènes ou plus, de 40 gènes ou plus, de 45 gènes ou plus, de 50 gènes ou plus, de 55 gènes ou plus, de 60 gènes ou plus, de 65 gènes ou plus, de 70 gènes ou plus, ou de 92 gènes ou plus.

15. Procédé conforme à la revendication 1, dans lequel ladite mesure comprend le fait de déterminer le niveau d'expression du Gène N° 1, en option à l'aide de la Séquence N° 1 utilisée comme sonde.

16. Procédé visant à déterminer le risque de développement de métastases d'un cancer de la prostate chez un sujet, lequel procédé comporte :
- le fait de mesurer, sur un échantillon comprenant des cellules cancéreuses de prostate issues dudit sujet, les niveaux d'expression de six gènes ou plus, choisis parmi les Gènes N° 1 à 362 indiqués dans les Figures 14 et 15,
- le fait de déterminer, en tant qu'indice global, la somme des niveaux d'expression desdits six gènes ou plus,
- et le fait de déterminer le risque de développement de métastases d'un cancer de la prostate chez ledit sujet, en se basant sur cet indice global,
étant entendu que les niveaux d'expression sont corrélés
soit à un faible risque de développement de métastases d'un cancer de la prostate,
soit à un haut risque de développement de métastases d'un cancer de la prostate.

17. Procédé conforme à l'une des revendications précédentes, dans lequel l'échantillon comprenant des cellules cancéreuses de prostate est une biopsie prélevée avec une aiguille ou une biopsie prélevée autrement.
